# EUROPEAN PATENT APPLICATION

(11) **EP 1 716 867 A1**
(43) Date of publication of application: **02.11.2006**
(21) Application number: 05710598.3
(22) Date of filing: 17.02.2005
(51) Int. Cl.: A61K 45/00, A61K 31/166, A61K 31/454, A61K 31/505, A61K 45/06, A61P 25/06, A61P 43/00, C07D 239/42, C07D 401/06

(54) **PREVENTIVES FOR MIGRAINE**

(30) Priority: 20.02.2004 JP 2004044089
(71) Applicant: Astellas Pharma Inc., Tokyo 103-8411 (JP)
(72) Inventor: AKUZAWA, Shinobu, c/o Astellas Pharma Inc., Tokyo 1038411 (JP); WATANABE, Toshihiro, c/o Astellas Pharma Inc., Tokyo 1038411 (JP)
(74) Representative: HOFFMANN EITLE
(86) International application number: PCT/JP2005/002946
(87) International publication number: WO 2005/079845

(57) **Abstract**

The present invention relates to prophylactic antimigraine agents comprising as an active ingredient a dual antagonist for the 5-HT_{2B} and 5-HT₇ receptors, the antagonist having a binding affinity selective for the 5-HT_{2B} and 5-HT₇ receptors.

Since these prophylactic antimigraine agents show an excellent pharmacological effect in comparison with the cases in which a 5-HT_{2B} receptor antagonist having a selective binding affinity to the 5-HT_{2B} receptor or a 5-HT₇ receptor antagonist having a selective binding affinity to the 5-HT₇ receptor is used alone, they are useful as drugs which are excellent in prophylaxis of migraine and in which the side effects found in the existing prophylactic antimigraine agents are reduced.

## Description

### Technical Field

This invention relates to pharmaceutical compositions useful as prophylactic antimigraine agents.

### Background of the Invention

Migraine is a periodically occurring pulsating headache which is a disease in which a strong pain occurs in one side or both sides of the head and is continued for several hours to about 3 days. It is suggested that the morbid state of this migraine advances by the following onset mechanism. Firstly, dura mater blood vessel once contracts by the action of 5-HT (serotonin) or the like neurotransmitter and then expands again, and in this case, inflammation advances by releasing CGRP or the like vasoactive peptide and plasma protein, thus resulting in the onset of headache.

The medicines targeting at migraine are divided into agents for prevention and agents for treatment. The former aims at reducing attack frequency by continuously administering them preventively before onset of the disease, and the latter aims at suppressing the pain by taking them after expression of the attack. Particularly as the preventive agents, Ca antagonists (e.g., lomerizine, flunarizine and the like), serotonin antagonists (e.g., pizotifen, methysergide and the like), β-adrenergic blocking drugs (e.g., propranolol and the like) and the like are clinically used in some country, but many side effects have been reported on all of them, and sufficient clinical effects have not been obtained yet.

Regarding the pizotifen as a serotonin antagonist among the aforementioned preventive agents, its efficacy is high in comparison with other agents, but there is a problem in that fatigue, sleepiness, giddiness, weight gain and the like side effects are found by its effective dose (J. Neurol. (1991) 238, S 45 - S 52). It is known that said compound has the affinity for all of the 5-HT receptor subtypes and also has high affinity for α₁, M₁, D₂ and the like various receptors.

5-HT is a monoamine neurotransmitter and expresses various physiological actions via a 5-HT receptor. The 5-HT receptor is classified into 7 families of from 5-HT₁ to 5-HT₇, and particularly in the case of 5-HT₂ receptor, 3 kinds of subtypes 5-HT_{2A}, 5-HT_{2B} and 5-HT_{2C} are known (Pharmacol. Rev. (1994) 46, 157 - 203). It has been suggested that this 5-HT is deeply concerned in the onset of migraine *(*Headache (1994) 34, 408 - 417). In addition, it has been reported that an agent having 5-HT receptor antagonism is effective in preventing migraine (Prog. Drug. Res. (1998) 51, 219 - 244).

In recent years, pharmacological studies on 5-HT receptor subtypes have been conducted. For example, it has been reported that a 5-HT_{2B} receptor antagonist inhibits leakage of protein into outside of guinea pig mCPP induced dural blood vessel (Cephalalgia (2003) 23, 117 - 123), and that the 5-HT_{2B} receptor localizing on vascular smooth muscle causes NO release, and the NO accelerates release of CGRP, substance P and the like nervous peptides from trigeminal nerve (J. Biol. Chem. (2000) 275, 9324 - 9331, Circ. Res. (1992) 70, 1313 - 1319). In addition, a result which suggests the effect to prevent migraine has been obtained by an animal model test using a compound (RS-127445) having selective binding affinity for the 5-HT_{2B} receptor (Clustar Headache and Related Conditions, vol. 9, edited by D. W. Bonhaus (England), Oxford University Press (1999), 278 - 286).

In addition, there are reports stating that 5-HT₇ receptor is distributed in the trigeminal nerve(Neurosci. Lett. (2001) 302, 9 - 12) and concerned in the vasodilation by 5-HT in the cerebrovascular smooth muscle, or concerned in the extra-dural blood vessel protein leakage acceleration action (Regional Anesth. (1996) 21, 219 - 225).

In another report, there is a description stating that 5-HT_{1D}, 5-HT_{2B} and 5-HT₇ receptors are present in dural blood vessel (FEBS Lett. (1995) 370, 215 - 221).

The relation between the affinity to the serotonin receptor subtypes and the prophylactic effect for migraine has been investigated using the existing non-selective serotonin antagonists. For example, it has been reported that the prophylactic effect is correlated with the affinity to the 5-HT_{2B} and 5-HT_{2C} receptors (non-patent literature 2). It has also been reported that, among the 8 subtypes of 5-HT_{1A} to 5-HT₇, 5-HT₇ has a high correlation (non-patent literature 3) and, among the 3 subtypes of 5-HT_{2B}, 5-HT_{2C} and 5-HT₇, 5-HT₇ has a high correlation (non-patent literature 4). However, since the test compounds employed in the reports are non-selective antagonistic compounds also having an effect for other receptors, it has not yet been identified which subtype is involved in prophylaxis of migraine.

On the other hand, U.S. Patent No. 6,440,988 (patent document 1) discloses a method of treating incontinence of urine in combination of a 5-HT_{2B} receptor-selective antagonistic compound with a 5-HT₇ receptor-selective antagonistic compound or by using a compound which has the both effects. However, there is no disclosure in the patent relative to migraine. International publication WO 03/000252 (patent document 2) describes that some compounds having an affinity to the 5-HT₆ and 5-HT₇ receptors are effective in treatment of a variety of central diseases including migraine. The compound disclosed in one example has an affinity to 5-HT₆, 5-HT₇ and 5-HT_{2B} receptors (page 20). However, there is no specific disclosure in the specification on the effect of the compounds in said patent application relative to prophylaxis of migraine.
(Non-patent literature 1)
   D.W. Bonhaus, Clustar Headache and Related Conditions, vol.9, (United Kingdom), Oxford University Press, 1999, p. 278-286
(Non-patent literature 2)
   Karin Schmuck, and 4 others, European Journal ofNeuroscience, (Holland), 1996, vol.8, p. 959-967
(Non-patent literature 3)
   Jose A. Terron, Proceedings of the Western Pharmacological Society, (USA) 1998, vol.41, p. 247-251
(Non-patent literature 4)
   Jose A. Terron, European Journal of Pharmacology, (Holland), 2002, vol.439, p. 1-11
(Patent Document 1) U.S. Patent No. 6,440,988 specification
(Patent Document 2) International publication WO 03/000252 pamphlet

### Disclosure of Invention

The present inventors worked assiduously to elucidate the relationship between an antagonist of the subtype of 5-HT receptors and the prophylactic effect for migraine, and as a result, they have found that among a wide variety of the subtypes, particularly the 5-HT_{2B} and 5-HT₇ receptors are important, and that practically the concomitant use of these selective antagonistic compounds results in great increase of the activity in comparison with that of single use, and further that the use of some compounds having both of the selective 5-HT_{2B} and 5-HT₇ receptor inhibitory activities has been confirmed to have the same effect. Thus, the present invention was completed. That is, the invention relates to a prophylactic antimigraine agent comprising as an active ingredient a selective dual antagonist for the 5-HT_{2B} and 5-HT₇ receptors. Since the prophylactic antimigraine agent of the invention have a weak antagonistic action on receptors other than the 5-HT_{2B} and 5-HT₇ receptors, they have reduced side effect caused by other receptors.

More particularly, the invention relates to (1) the above prophylactic antimigraine agents, in which the selective dual antagonist for the 5-HT_{2B} and 5-HT₇ receptors comprises a) a 5-HT_{2B} receptor antagonistic compound as a first ingredient having a selective binding affinity to the 5-HT_{2B} receptor, and b) a 5-HT₇ receptor antagonistic compound as a second ingredient having a selective binding affinity to the 5-HT₇ receptor; and (2) the above prophylactic antimigraine agents, in which the selective dual antagonist for the 5-HT_{2B} and 5-HT₇ receptors comprises a dual antagonist for the 5-HT_{2B} and 5-HT₇ receptors having a selective binding affinity to both of the 5-HT_{2B} and 5-HT₇ receptors.

In addition, the invention encompasses a combined prophylactic preparation for migraine which comprises a) a first pharmaceutical preparation containing as an active ingredient a 5-HT_{2B} receptor antagonistic compound having a selective binding affinity to the 5-HT_{2B} receptor, and b) a second pharmaceutical preparation containing as an active ingredient a 5-HT₇ receptor antagonistic compound having a selective binding affinity to the 5-HT₇ receptor, and of which the first and second preparations are administered simultaneously or separately.

In addition, the invention includes the following embodiment.
[1] Use of the selective dual antagonist for the 5-HT_{2B} and 5-HT₇ receptors for the manufacture of a prophylactic antimigraine agent.
[2] Use of "a 5-HT_{2B} receptor antagonistic compound having a selective binding affinity to the 5-HT_{2B} receptor" for the manufacture of a prophylactic antimigraine agent comprising as an active ingredient a selective dual antagonist for the 5-HT_{2B} and 5-HT₇ receptors.
[3] Use of "a 5-HT₇ receptor antagonistic compound having a selective binding affinity to the 5-HT₇ receptor" for the manufacture of a prophylactic antimigraine agent containing as an active ingredient a selective dual antagonist for the 5-HT_{2B} and 5-HT₇ receptors.
[4] A method for prophylaxis of migraine which comprises administering a therapeutically effective amount of a selective dual antagonist for the 5-HT_{2B} and 5-HT₇ receptors to a patient.
[5] A method for prophylaxis of migraine which comprises administering a combination comprising a pharmaceutical preparation containing as an active ingredient a 5-HT_{2B} selective receptor antagonistic compound and a pharmaceutical preparation containing as an active ingredient a 5-HT₇ receptor selective antagonistic compound, simultaneously or separately to a patient.
[6] An enhancer of the prophylactic effect for migraine in a patient who has received administration of "a 5-HT₇ receptor antagonistic compound having a selective binding affinity to the 5-HT₇ receptor" for the purpose of prophylaxis of migraine, which comprises as an active ingredient "a 5-HT_{2B} receptor antagonistic compound having a selective binding affinity to the 5-HT_{2B} receptor".
[7] An enhancer of the prophylactic effect for migraine in a patient who has received administration of "a 5-HT2B receptor antagonistic compound having a selective binding affinity to the 5-HT_{2B} receptor" for the purpose of prophylaxis of migraine, which comprises as an active ingredient "a 5-HT₇ receptor antagonistic compound having a selective binding affinity to the 5-HT₇ receptor".

### Brief Description of Drawings

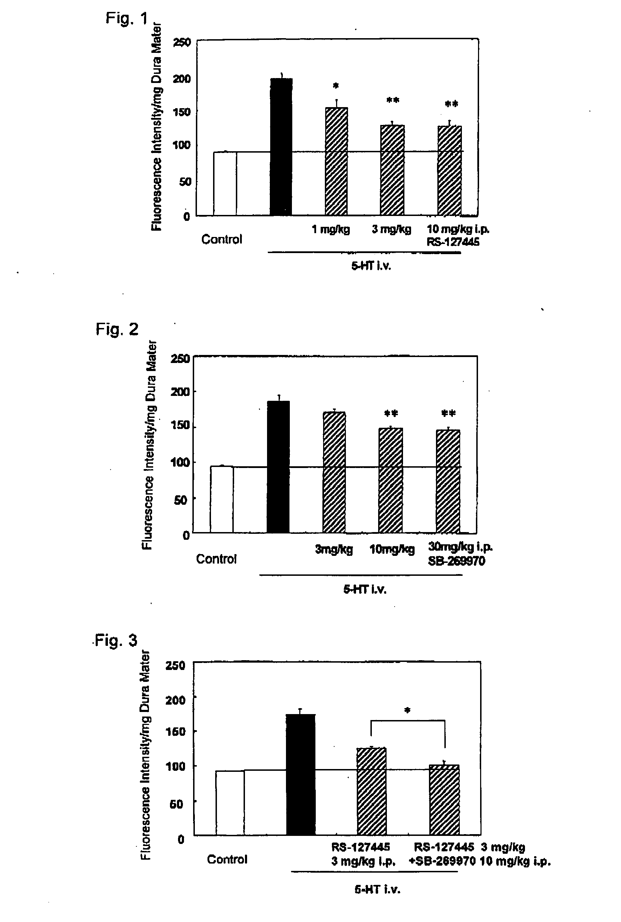

Fig. 1 shows a graph which indicates the results of measurement of the amount of leaked proteins after administration of RS-127445 in a migraine model of guinea pig in the test method (4). Statistical testing was made by the Dunnett's test; * indicates probability value is less than 5%, and ** indicates that is less than 1%.
Fig. 2 shows a graph which indicates the results of measurement of the amount of leaked proteins after administration of SB-269970 in a migraine model of guinea pig in the test method (4). Statistical testing was made by the Dunnett's test; ** indicates probability value is less than 1%.
Fig. 3 shows a graph which indicates the results of measurement of the amount of leaked proteins after simultaneous administration of RS-127445 and SB-269970 in a migraine model of guinea pig in the test method (4). Statistical testing was made by the T test; * indicates probability value is less than 5%.

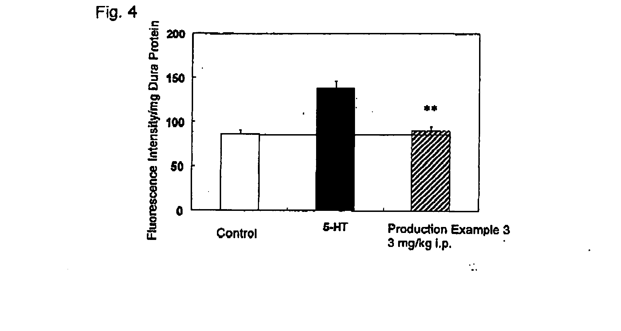

Fig. 4 shows a graph which indicates the results of measurement of the amount of leaked proteins after administration of the compound of Example 3 in a migraine model of guinea pig in the test method (4). Statistical testing was made by the T test; * indicates probability value is less than 5%.

### Best Mode for Carrying Out the Invention

The invention will be explained in detail hereinafter.

In this specification, the term "a prophylactic antimigraine agent" means pharmaceutical formulations or pharmaceutical compositions which are prescribed for a migraine patient or a patient who has been diagnosed to be migraine and in whom periodical attacks of migraine occur, and which are administered before the attack in order to reduce the frequency of attack or the degree of pain.

The term "antagonist" means a drug which acts antagonistically on an agonist to reduce the effect. In the present invention, a "dual antagonist for the 5-HT_{2B} and 5-HT₇ receptors" means a drug which acts antagonistically with serotonin to reduce simultaneously the effect mediated by both of the 5-HT_{2B} and 5-HT₇ receptors, and includes pharmaceutical preparations containing as an active ingredient a compound having both of the antagonistic actions, and pharmaceutical preparations containing two active ingredients, i.e., a compound having a 5-HT_{2B} receptor antagonistic action and a compound having a 5-HT₇ receptor antagonistic action.

The term "binding affinity" means the ability capable of binding a part of a receptor, and it can be assessed by comparing the Ki values calculated by an in vitro receptor-binding test as described below in Test Example, or in some cases by comparing the IC₅₀ values in the receptor-binding test conducted in the parallel condition. In this context, when the IC₅₀ value cannot be calculated because a sufficient inhibitory effect is not indicated at a given concentration in the receptor-binding test, the IC₅₀ value is sometimes regarded as over the concentration.

The terms "5-HT_{2B} receptor antagonistic compound", "5-HT₇ receptor antagonistic compound" and "dual antagonistic compound of the 5-HT_{2B} and 5-HT₇ receptors" include preferably antagonistic compounds in which the receptor binding affinity Ki values in the respectively selectively binding receptors are 1µM or lower, more preferably 0.5µM or lower, even more preferably 0.1µM or lower, and particularly 0.05µM or lower.

When the binding affinity to a certain receptor is more "selective" than that of other receptors, it means that the binding affinity to said receptor is higher than that of "other receptors". In the invention, the term "selective" means that the Ki value or IC₅₀ value indicating the binding affinity to said receptor is one-tenth or less, preferably one-fiftieth or less, more preferably one-hundredth or less, even more preferably one-five hundredth or less, particularly one-thousandth or less in comparison with that of "other receptors".

In this context, "other receptor" includes other receptors reported in the existing non-selective serotonin antagonists, which are particularly involved in unfavorable effects,

Thus, the "5-HT_{2B} receptor antagonistic compound having a selective binding affinity to the 5-HT_{2B} receptor (hereinafter abbreviated to 5-HT_{2B} selective antagonistic compound)" includes specifically those selective against α₁, M₁ and D₂ receptors, preferably against α₁, M₁, D₂, 5-HT_{1A}, 5-HT_{1B}, 5-HT₄, 5-HT₆ and 5-HT₇ receptors, more preferably against α₁, M₁, D₂, 5-HT_{1A}, 5-HT_{1B}, 5-HT_{2A}, 5-HT_{2C}, 5-HT₃, 5-HT₄, 5-HT₆ and 5-HT₇ receptors.

The "5-HT₇ receptor antagonistic compound having a selective binding affinity to the 5-HT₇ receptor (hereinafter abbreviated to 5-HT₇ selective antagonistic compound)" includes those selective against the α₁, M₁ and D₂ receptors, preferably against α₁, M₁, D₂, 5-HT_{1A}, 5-HT_{1B}, 5-HT_{2B}, 5-HT₃, 5-HT₄ and 5-HT₆ receptors, more preferably against α₁, M₁, D₂, 5-HT_{1A}, 5-HT_{1B}, 5-HT_{2A}, 5-HT_{2B}, 5-HT_{2C}, 5-HT₃, 5-HT₄ and 5-HT₆ receptors.

The "dual antagonistic compound having a selective binding affinity to both the 5-HT_{2B} and 5-HT₇ receptors (hereinafter abbreviated to 5-HT_{2B} and 5-HT₇ selective dual antagonistic compound)" includes those selective against the α₁, M₁ and D₂ receptors, preferably against α₁, M₁, D₂, 5-HT_{1A}, 5-HT_{1B}, 5-HT₃, 5-HT₄ and 5-HT₆ receptors, more preferably against α₁, M₁, D₂, 5-HT_{1A}, 5-HT_{1B}, 5-HT_{2A}, 5-HT_{2C}, 5-HT₃, 5-HT₄ and 5-HT₆ receptors.

In addition, the "a selective dual 5-HT_{2B} and 5-HT₇ antagonist" includes "combined preparations comprising a 5-HT_{2B} selective antagonistic compound and a 5-HT₇ selective antagonistic compound" or "a dual antagonist for the 5-HT_{2B} and 5-HT₇ receptors comprising as an active ingredient a 5-HT_{2B} and 5-HT₇ selective dual antagonistic compound ", in which the binding affinity (Ki value or IC₅₀ value) to the 5-HT_{2B} and 5-HT₇ receptors is one-tenth or less, preferably one-fiftieth or less, more preferably one-hundredth or less, even more preferably one-five hundredth or less, and particularly one-thousandth or less, to the α₁, M₁ and D₂ receptors, preferably to α₁, M₁, D₂, 5-HT_{1A}, 5-HT_{1B}, 5-HT₃, 5-HT₄ and 5-HT₆ receptors, more preferably to α₁, M₁, D₂, 5-HT_{1A}, 5-HT_{1B}, 5-HT_{2A}, 5-HT_{2C}, 5-HT₃, 5-HT₄ and 5-HT₆ receptors.

The "5-HT_{2B} selective antagonistic compound", "5-HT₇ selective antagonistic compound" and "5-HT_{2B} and 5-HT₇ selective dual antagonistic compound" can easily be found by screening a large amount of compounds to determine receptor affinity according to a method as shown in Reference Examples. In this evaluation, an HTS (high-throughput screening) method is employed as a routine and efficient means. As for the test compounds employed in screening, new synthetic compounds, commercially available products or known compounds registered in chemical libraries on which a variety of activities are unknown, and a group of compounds obtained by a combinatorial chemical technique, can be used. In addition, naturally occurring products derived from culture supernatants of microorganisms, plants or marine organisms, or animal tissue extracts, and the like can be employed. In addition, chemically modified compounds derived from compounds found in screening can be used.

The "5-HT_{2B} selective antagonistic compound" of the invention can be found by accomplishing a receptor affinity screening method as described in Reference Examples 1 and 3 below or a similar method thereto. The compounds, specifically, includes for example known 5-HT_{2B} selective antagonistic compounds, RS-127445 (British Journal of Pharmacology (1999) 127, 1075-1082), LY-266097 (J. Serotonin Res. (1996) 3, 131), SB-200646 (J. Med. Chem. (1993) 36,1104), SB-204741 (J. Med. Chem. (1995) 38, 855), SB-206553 (J. Med. Chem. (1996) 39, 2773), SB-221284 (9th RSC-SCI Medicinal Chemistry Symposium (1997) P1 (Poster), 7 Sep), EGIS-7625 (Cardiovascular Drugs and Therapy (2003) 17, 427-434, 4-(thio or selenoxanthen-9-ylidene)piperidine or acridine derivatives (US2003166672), 2-oxazoleamine derivatives (WO2003068226), 2-thiazole-amine derivatives (WO2003068227), and the like, as far as they are 5-HT_{2B} receptor selective compounds.

The "5-HT₇ selective antagonistic compound" of the invention can be found by accomplishing a receptor affinity screening method as described in Reference Examples 2 and 3 below or a similar method thereto. The compounds, specifically, includes for example known 5-HT₇ selective antagonistic compounds, DR-4004 (J. Med. Chem. (1999) 42, 533), SB-269970 (J. Med. Chem. (2000) 43, 342-345), SB-691673 (Bioorg. Med. Chem. (2003) 13, 1055-1058), aminotriazole derivatives (Bioorg. Med. Chem. (2004) 14, 4245-4248), aminotetralin derivatives (J. Med. Chem. (2004) 47, 3927-3930), aminochromane derivatives (J. Med. Chem. (2004) 47, 3927-3930), 11-phenylapomorphine derivatives (J. Med. Chem. (2001) 44,1337-1340) and the like, as far as they are 5-HT₇ receptor selective compounds.

In addition, the " 5-HT_{2B} and 5-HT₇ selective dual antagonistic compound" can easily be found by successively accomplishing a receptor affinity screening method as described in Reference Examples 1 to 3. For example, the compounds represented by the following general formula (I) or their salts are exemplified as specific compounds. 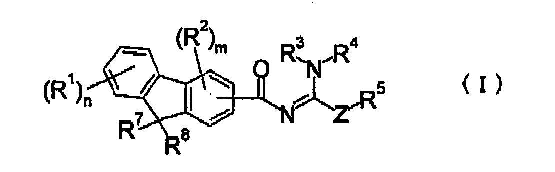 wherein the symbols have the following significances.
R¹ and R² are the same or different from each other and each represents -R°, lower alkenyl, lower alkynyl, halogen, -OH, -O-R⁰, -O-CO-R⁰, -NH₂, -NR⁶-R⁰, -CN, -NO₂, -CHO, -CONH₂, -CO-NR⁶-R⁰, -CO₂H, -CO₂-R⁰, -CO-R⁰, -NR⁶-CO-R⁰, -NR⁶-CO₂-R⁰, -O-CO-NR⁶-R⁰, -SH, -S(O)ₚ-R⁰, -S(O)₂-NH₂, -S(O)₂-NR⁶-R⁰, -NR⁶-S(O)₂-R⁰, -R⁰⁰-O-CO-R⁰, -R⁰⁰-NR⁶-R⁰, -R⁰⁰-CN, -R⁰⁰-CONH₂, -R⁰⁰-CO-NR⁶-R⁰, -R⁰⁰-CO₂H, -R⁰⁰-CO₂-R⁰, -R⁰⁰-CO-R⁰, -R⁰⁰-NR⁶-CO-R⁰, -R⁰⁰-NR⁶-CO₂-R⁰, -R⁰⁰-O-CO-NR⁶-R⁰, cycloalkyl or nitrogen-containing saturated heterocyclic ring; wherein the nitrogen-containing saturated heterocyclic ring may be substituted with 1 to 2 substituents selected from the group consisting of lower alkyl, -OH, -O-R⁰, -NH₂, -NR⁶-R⁰ and oxo (=O);
R° is the same or different from each other and represents a lower alkyl which may be substituted with 1 or more substituents selected from the group consisting of -OH, -O-C₁₋₄ alkyl, -NH₂, -NR⁶-C₁₋₄ alkyl and halogen;
R⁶ is the same or different from one another and represents lower alkyl or H;
R⁰⁰ is the same or different from one another and represents lower alkylene;
p is 0, 1 or 2;
n is 0, 1 or 2;
m is 0 or 1;
R⁷ and R⁸ are the same or different from each other and each represents -H, -R⁰, halogen, -OH, -O-R⁰, -NH₂, -NR⁶-R⁰, -NR⁶-CO-R⁰, -O-R⁰⁰-OH, -O-R⁰⁰-O-R⁰, cycloalkyl, nitrogen-containing saturated heterocyclic ring, or R⁷ and R⁸ may be taken together to form a group selected from the group consisting of oxo (=O), =N-OH, =N-OR⁰ and tetrahydropyranylidene, or R⁷ and R⁸ may be taken together to form a lower alkylene which may be interrupted by 1 to 2 groups selected from the group consisting of -O-, -S(O)ₚ-, -NR⁶- and -CONR⁶-, and may form a 3- to 8-membered ring together with the C atom to which they are attached.
Z is -NH-;
R³ is -H or R⁰; and
R⁴ and R⁵ are the same or different from each other and each represents -H, -R⁰, -CO₂-R⁰, -CO-R⁰, or R⁴ and R⁵ may be taken together to form a divalent group, which may form a 5-membered heterocyclic ring together with the -N-C-Z- group to which R⁴ and R⁵ are attached, wherein Z may further be -O- or S- and the 5-membered, ring may be substituted with 1 or 2 substituents selected from lower alkyl, -OH, -O-R⁰, -NH₂, -NR⁶-R⁰, and oxo (=O); the same is applied hereinafter.

In this context, the term "lower" as defined in the above-mentioned general formula, unless otherwise indicated, means a straight or branched carbon chain of 1 to 6 carbon atoms (hereinafter abbreviated to as C₁₋₆). Thus, the "lower alkyl" means an alkyl of the carbon number C₁₋₆, including preferably methyl, ethyl, propyl, isopropyl, butyl, isobutyl, tert-butyl.

The "lower alkenyl" includes C₂₋₆ alkenyl groups, preferably, vinyl, allyl, 1-propenyl, isopropenyl, 1-butenyl, 2-butenyl and 3-butenyl. The "lower alkynyl" includes C₂₋₆ alkynyl groups, preferably, ethynyl, 1-propynyl, 2-propynyl, 1-butynyl, 2-butynyl, 3-butynyl and 1-methyl-2-propynyl.

The "lower alkylene" includes, preferably, straight chain alkylene such as methylene, ethylene, trimethylene, tetramethylene and the like, and branched alkylene such as methylmethylene, Methylene, ethylene and trimethylene are particularly preferred.

The "halogen" means F, Cl, Br or I.

The "cycloalkyl" means C₃₋₁₀ cycloalkyl group which may have a bridge, and includes preferably cyclopropyl, cyclopentyl and cyclohexyl groups.

The "nitrogen-containing saturated heterocyclic ring" means a 5- to 8-membered saturated or partially unsaturated monocyclic heterocycle which contain one N atom and may contain an additional heteroatom selected from N, S and O, and includes preferably, pyrrolidinyl, piperidinyl, piperazinyl, azepanyl, diazepanyl, morpholinyl, thiomorpholinyl and tetrahydropyridyl groups.

The "oxygen-containing saturated heterocyclic ring" means a 5- to 8-membered saturated or partially unsaturated monocyclic heterocycle which contain one O atom and may contain an additional N atom, and includes preferably, tetrahydrofuranyl, tetrahydropyranyl, dihydropyranyl and morpholinyl groups.

The term "which may be substituted" indicates "unsubstituted" or "having 1 to 5 identical or different substituents".

For example, the term "lower alkyl which may be substituted with halogen" means the above-mentioned lower alkyl and lower alkyl substituted with one or more halogens, preferably C₁₋₂ alkyl having 1 to 5 F atoms, more preferably fluoromethyl, difluoromethyl, trifluoromethyl.

The term "lower alkylene which may be interrupted by 1 to 2 groups selected from the group consisting of -O-, -S(O)ₚ-, -NR⁶- and -CONR⁶-" means a lower alkylene or lower alkylene into which 1 or 2 groups selected from the group consisting of -O-, -S(O)ₚ-, -NR⁶- and -CONR⁶- is inserted at the internal or terminal position. For example, it is exemplified by -(CH₂)₂-, -(CH₂)₃-, -(CH₂)₄-, -(CH₂)₃-O-, -(CH₂)₂-O-(CH₂)₂-, -(CH₂)₂-S(O)-(CH₂)₂-, -(CH₂)₂-N(CH₃)-(CH₂)₂-, -O-(CH₂)₂-O-, -S-(CH₂)₂-S-, -CH₂-S-CH₂-, or CH₂CONHCH₂-, preferably by -(CH₂)₄-, -S-(CH₂)₂-S-, -(CH₂)₂-O-(CH₂)₂-, or -(CH₂)₃-O-.

The salts of the compounds as active ingredients in the drugs of the invention are pharmaceutically acceptable salts, specifically including acid addition salts with inorganic acids such as hydrochloric acid, hydrobromic acid, hydroiodic acid, sulfuric acid, nitric acid, phosphoric acid, and the like, or with organic acids such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, aspartic acid, glutamic acid, and the like. In addition, salts with inorganic bases including metals such as sodium, potassium, magnesium, calcium, aluminum, and the like, or organic bases such as methylamine, ethylamine, ethanolamine, lysine, ornithine, and the like, and ammonium salts are included.

In some cases, the compound as an active ingredient in the drugs of the invention exists as a geometrical isomer or tautomer. For example, the following tautomers exist in a compound of the above-mentioned formula (I) in which R³ is -H.

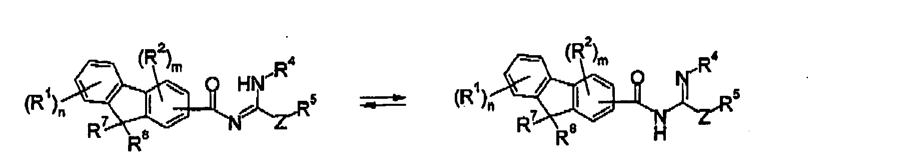

The active ingredient in the drugs of the present invention includes one of such tautomers or a mixture thereof. In addition, when an isomer exist on the basis of an asymmetric carbon atom or atoms, the active ingredient in the drugs of the invention includes these optical isomers in a form of mixture or isolated form. In some cases, N-oxides may be formed depending on the type of the substituents; such N-oxide derivatives are included in the invention. In addition, a variety of their hydrates or solvates and polymorphic substances are also included.

In the compounds as active ingredients in the drugs of the invention, some compounds are metabolized in the living body and converted into the compounds of formula (I) or their salts; such compounds, so-called prodrugs, are also included in the invention. As the groups for forming prodrugs, those described in Prog. Med. 5:2157-2161 (1985) or in "Iyakuhin no Kaihatsu (Development of Medicines)" vol.7, Bunshi Sekkey (Molecular Design) 163-198, published in 1990, Hirokawa Publishing Company, are exemplified.

Typical processes for producing the compounds as active ingredients in the drugs of the invention will be illustrated as follows. The 5-HT_{2B} selective antagonistic compounds and 5-HT₇ selective antagonistic compounds may be produced referring to the following documents.

### [5-HT_{2B} selective antagonistic compounds]

RS-127445: WO09744326; LY-266097: J. Med. Chem. (1996) 39,2773-2780; SB-200646: J. Med. Chem. (1993) 36,1104; SB-204741: J. Med. Chem. (1995) 38, 855; SB-206553: J. Med. Chem. (1996) 39,2773; EGIS-7625: WO199744334; 4-(thio or selenoxanthen-9-ylidene)piperidine or acridine derivatives: US2003166672; 2-oxazoleamine derivatives: WO2003068226; 2-thiazoleaminc derivatives: W02003068227.

### [5-HT₇ selective antagonistic compounds]

DR-4004: J. Med. Chem. (1999) 42, 533; SB-269970: J. Med. Chem. (2000) 43, 342-345; SB-691673: Bioorg. Med. Chem. (2003) 13, 1055-1058; aminotriazole derivatives: Bioorg. Med. Chem. (2004) 14, 4245-4248; aminotetraline derivatives: J. Med. Chem. (2004) 47, 3927-3930; aminochromane derivatives: J. Med. Chem. (2004) 47, 3927-3930; 11-phenylapomorphine derivatives: J. Med. Chem. (2001) 44, 1337-1340.

The compounds represented by formula (I) may be produced according to the following processes. 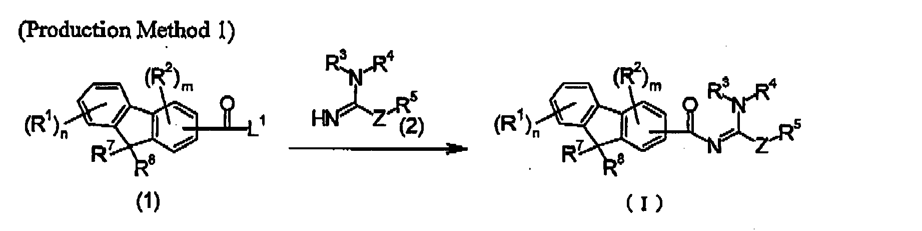 (wherein L¹ represents a leaving group such as -OH or -O-lower alkyl, or halogen, -O-methanesulfonyl or -O-p-toluenesulfonyl, or the like)

The compounds represented by formula (I) (hereinafter abbreviated to as Compound (I)) can be produced by subjecting a carboxylic acid or its reactive derivative represented by (1) and an amine derivative (2) to an amidation reaction. The amidation reaction may be carried out according to the method as described for example in "Seikagaku Jikken Kohza (Manual of Biochemical Experiments)" vol.1, Tanpakushitu no Kagaku (Chemistry of Proteins) IV, published in 1977, Tokyo Kagaku Dozin Co., Ltd.

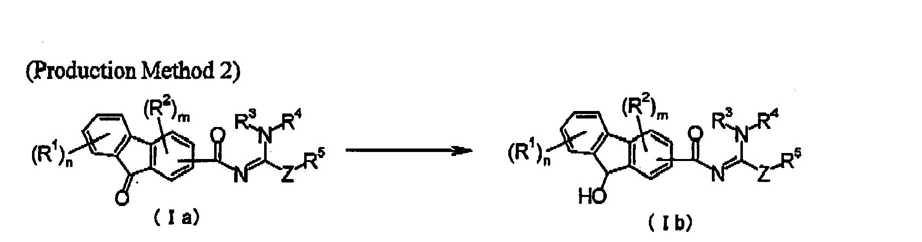

Compounds (I) in which -CR⁷R⁸- is -CH(OH)-, i.e. Compounds (Ib) may be produced by reducing Compounds (Ia)(in which the corresponding moiety is a carbonyl group) of the invention. The reduction may be conducted according to the method as described for example in Comprehensive Organic Transformation (1989), VHC Publishers, Inc.

Thus resulting compounds may be isolated and purified in a free form or as a salt by conventional salt-forming treatment. Isolation and purification may be achieved by applying a conventional chemical procedure such as extraction, concentration, distillation, crystallization, filtration, recrystallization, a variety of chromatography, and so on.

A variety of isomers can be isolated by a conventional way utilizing difference of the physicochemical properties between the isomers. For example, the optical isomers may be separated and purified by formation of diastereomeric salts from the racemates with an optically active organic acid (e.g., tartaric acid) and subsequent fractional recrystallization, or by column chromatography using a chiral stationary phase. In addition, the optically active compounds can be produced using a suitable optically active compound as a starting material. In this connection, a mixture of diastereomers may also be separated by fractional crystallization or chromatography.

In a "combination for prophylaxis for migraine", the term "combination" means a set of pharmaceutical preparations which contains each ingredient independently and can be used in combination therapy; the combined preparation may be marketed as a combined packaged product (e.g., form of kit) or each preparation independently may be made a sale for combined administration. In this connection, the term "simultaneously" means that the first preparation and the second preparation are administered together, and the term "separately" means that the first preparation and the second preparation are administered separately via the same or different route or by the same or different frequency or interval of administration. Preferably, each pharmaceutical preparation may be administered simultaneously or separately according to a prescription of formulation suitable for the respective preparations and to the condition of administration such as route and frequency of administration, considering bioavailability and stability of each preparation. When the duration of the active ingredients in the first and second preparations is approximately the same, it is appropriate to administer these preparations simultaneously or within about 1 hour. In a case of simultaneous administration, the separately formulated preparations may be mixed with a diluent just before use and then administered.

The kit comprises the first preparation containing 5-HT_{2B} selective antagonistic compound and the second preparation containing 5-HT₇ selective antagonistic compound, and if required it may be made into a package product which may contain an additional preparation (e.g., placebo) or specification material making administration on schedule easy.

In the present invention, the prophylactic antimigraine agents comprising as an active ingredient a selective dual antagonist for the 5-HT_{2B} and 5-HT₇ receptors as well as the above-mentioned first and second preparations constituting the combined preparation of the invention, may be prepared by a conventional method using carriers or fillers for pharmaceuticals usually employed in this field. Administration may be conducted orally as tablets, pills, capsules, granules, powders, liquids and solutions, and the like, or parenterally as intravenous or intramuscular injections or as external preparations such as ointments, plasters, creams, jellies, pups, aerosol, lotions, ophthalmic solutions, ophthalmic ointments, and the like or as suppositories or inhalations.

As the solid compositions for oral administration in the invention, tablets, powders, granules and the like are used. In such solid compositions, one or more of active substances are mixed with at least one inert excipient, for example, lactose, mannitol, glucose, hydroxypropylcellulose, microcrystalline cellulose, starch, polyvinylpyrrolidone, magnesium metasilicate aluminate, and the like. The compositions may contain inert additives, for example, lubricants such as magnesium stearate, disintegrators such as carboxymethyl starch sodium, or solubilizing agents. The tablets or pills if required may be coated with a sugar-coating agent or a gastric or enteric coating agent.

The liquid compositions for oral administrations include pharmaceutically acceptable emulsions, liquids and solutions, suspensions, syrups, elixirs, and the like, in which an inert solvent, for example, purified water, ethanol, and the like may be used. In addition to the inert solvent, the composition may contain auxiliary agents such as solubilizing agent, wetting agent, or suspending agent, sweeteners, correctives, flavors, or antiseptics.

The injection preparations for parenteral administration include sterile aqueous or non-aqueous liquids and solutions, suspensions, and emulsions. The aqueous solvent includes, for example, distilled water for injection and physiological saline. The non-aqueous solvent includes, for example, propylene glycol, polyethylene glycol, a vegetable oil such as olive oil, an alcohol such as ethanol, polysorbate 80 (name in Japanese Pharmacopoeia), and the like. Such compositions may contain further isotonic agents, antiseptics, wetting agents, emulsifying agents, dispersants, stabilizers, and solubilizing agents. These may be sterilized for example by filtration through a bacterial filter or combination with a bactericide or by irradiation. These may also be prepared as sterile solid compositions and dissolved or suspended in sterilized water or sterile solvent for injection just before use.

The prophylactic antimigraine agents of the invention is usually administered prophylactically before attack of migraine. Thus, it is desired to continuously take them during high frequency of migraine attack. When the prophylactic antimigraine agents of the invention is an antagonist which comprises the first ingredient "5-HT_{2B} selective antagonistic compound" and the second ingredient "5-HT₇ selective antagonistic compound", the amount of combination of each compound may properly be determined according to the condition of individual patients within the range of clinically effective amount of each compound prescribed as a single unit.

When the prophylactic antimigraine agent of the invention is an antagonist which comprise the 5-HT_{2B} and 5-HT₇ selective dual antagonistic compound, they may be administered orally at a daily dose of about 0.001 to 50 mg/kg (body weight), preferably 0.01-30 mg/kg, more preferably 0.05-10 mg/kg, or intravenously at a daily dose of about 0.0001 to 10 mg/kg (body weight), preferably 0.001-1.0 mg/kg, in a single or divided doses a day. The dosage may suitably be determined depending on an individual case considering the condition, age, sex, and so on.

The dosage of each compounds contained as active ingredients in the above-mentioned first and second preparations constituting the combination of the invention may suitably be determined according to the condition of individual patients within the range of clinically effective amount of each compound prescribed as a single preparation.

### Preparations

The following describes concrete preparations of the compounds described in formula (I) which is active ingredients in the drugs of the invention. In this connection, production methods of starting compounds are described as Reference Preparations.

### Reference Preparation 1-a

Diethyl 2'-methylbiphenyl-2,4-dicarboxylate was obtained by allowing 4-bromoisophthalic acid diethyl ester to react with 2-methylphenylboronic acid, sodium carbonate and tetrakistriphenylphosphine palladium under heating in toluene-ethanol-water. FAB-MS: 313 (M + H)⁺.

### Reference Preparation 1-b

2'-Methylbiphrenyl-2,4-dicarboxylic acid was obtained by treating ethanol solution of diethyl 2'-methylbiphenyl-2,4-dicarboxylate with 1 M sodium hydroxide.
FAB-MS: 257 (M + H)⁺.

### Reference Preparation 1-c

5-Methyl-9-oxo-9H-fluorene-2-carboxylic acid was obtained by heating 2'-methylbiphenyl-2,4-dicarbaxylic acid in polyphosphoric acid. FAB-MS: 239 (M + H)⁺.

### Reference Preparation 2

3'-Methylbiphenyl-2,4-dicarboxylic acid was treated in the same manner as in Reference Preparation 1-c, and then the thus obtained solid was heated in ethanol in the presence of concentrated sulfuric acid to carry out esterification. After treatment of the reaction, this was separated and purified by a silica gel column chromatography to obtain ethyl 6-methyl-9-oxo-9H-fluorene-2-carboxylate [FAB-MS: 267 (M + H)⁺] and ethyl 8-methyl-9-oxo-9H-fluorene-2-carboxylate [FAB-MS: 267 (M + H)⁺], respectively.

### Reference Preparation 3

In the same manner as in Reference Preparation 1-a, ethyl 3-chloro-2'-formylbiphenyl-4-carboxylate was produced from ethyl 4-bromo-2-chlorobenzoate and 2-formylphenylboronic acid. FAB-MS: 288 (M)⁺.

3'-Chloro-4'-(ethoxycarbonyl)biphenyl-2-carboxylic acid was obtained by allowing ethyl 3-chloro-2'-formylbiphenyl-4-carboxylate to react with sodium perchlorate, sodium dihydrogenphosphate and 2-methyl-2-butene in tert-butanol-acetonitrile-water at room temperature. FAB-MS: 305 (M + H)⁺.

Thereafter, in the same manner as in Reference Preparation 1-c and Reference Preparation 2, ethyl 1-chloro-9-oxo-9H-fluorene-2-carboxylate [FAB-MS. 287 (M + H)⁺] and ethyl 3-chloro-9-oxo-9H-fluorene-2-carboxylate [FAB-MS: 287 (M + H)⁺] were produced, respectively.

### Reference Preparation 4-a

9-Hydroxy-9-methyl-9H-fluorene-2-carboxylic acid was obtained by allowing 9-oxo-9H-fluorene-2-carboxylic acid to react with methyl lithium in THF at from -20°C to
0°C. FAB-MS: 239 (M - H)⁻.

### Reference Preparation 4-b

Methyl 9-hydroxy-9-methyl-9H-fluorene-2-carboxylate was obtained by allowing 9-hydroxy-9-methyl-9H-fluorene-2-carboxylic acid to react with sodium bicarbonate and methyl iodide in DMF at room temperature. FAB-MS: 255 (M + H)⁺.

### Reference Preparation 4-c

Methyl 9-methoxy-9-methyl-9H-fluorene-2-carboxylate was obtained by allowing methyl 9-hydroxy-9-methyl-9H-fluorene-2-carboxylate to react with iron nitrate in methanol under heating. FAB-MS: 269 (M + H)⁺.

### Reference Preparation 5

Methyl 9-methoxymethyl-9-methyl-9H-fluorene-2-carboxylate was obtained by allowing methyl 9-hydroxy-9-methyl-9H-fluorene-2-carboxylate to react with sodium hydride and methoxymethyl chloride in DMF at room temperature. FAB-MS: 298 (M)⁺.

### Reference Preparation 6-a

In THF, methylmagnesium bromide was allowed to act upon 3-chloropropan-1-ol to form magnesium oxide, and then magnesium metal was allowed to react therewith, thereby preparing a Grignard reagent (ClMg(CH₂)₃OMgBr). This was allowed to react with 9-oxo-9H-fluorene-2-carboxylic acid in the same manner as in Reference Preparation 4-a, and then the thus obtained 9-hydroxy-9-hydroxypropyl-9H-fluorene-2-carboxylic acid was allowed to react with methyl iodide in the same manner as in Reference Preparation 4-b, thereby obtaining methyl 9-hydroxy-9-hydroxypropyl-9H-fluorene-2-carboxylate.
FAB-MS: 297 (M - H)⁻.

### Reference Preparation 6-b

Methyl 4',5'-dihydro-3'H-spiro[fluorene-9,2'-furan]-2-carboxylate was obtained by allowing methyl 9-hydroxy-9-hydroxypropyl-9H-fluorene-2-carboxylate to undergo the reaction under heating in toluene in the presence of p-toluenesulfonic acid. FAB-MS: 281 (M+H)⁺.

### Reference Preparation 7-a

Methyl 8-bromomethyl-9-oxo-9H-fluorene-2-carboxylate was obtained by allowing methyl 8-methyl-9-oxo-9H-fluorene-2-carboxylate to react with N-bromosuccinimide and 2,2'-azobisisobutyronitrile under heating in carbon tetrachloride.
EI-MS: 330 (M)⁺, 332 (M + 2)⁺.

### Reference Preparation 7-b

Methyl 8-dimethylaminomethyl-9-oxo-9H-fluorene-2-carboxylate was obtained by allowing methyl 8-bromomethyl-9-oxo-9H-fluorene-2-carboxylate to react with dimethylamine (2 M, methanol solution) and potassium carbonate at room temperature in
THF. FAB-MS: 296 (M + H)⁺.

### Reference Preparation 8-a

Methyl 8-acetoxymethyl-9-oxo-9H-fluorene-2-carboxylate was obtained by allowing methyl 8-bromomethyl-9-oxo-9H-fluorone-2-carboxylate to react with potassium acetate at room temperature in DMF. FAB-MS: 311 (M + H)⁺.

### Reference Preparation 8-b

Methyl 8-hydroxymethyl-9-oxo-9H-fluorene-2-carboxylate was obtained by allowing methyl 8-acetoxymethyl-9-oxo-9H-fluorene-2-carboxylate to react with potassium carbonate at room temperature in methanol-THF. FAB-MS: 269 (M + H)⁺.

### Reference Preparation 8-c

Methyl 8-methoxymethyl-9-oxo-9H-fluorene-2-carboxylate was obtained by allowing methyl 8-hydroxymethyl-9-oxo-9H-fluorene-2-carboxylate to react with methyl iodide and silver oxide under heating in acetonitrile. FAB-MS: 283 (M + H)⁺.

### Reference Preparation 9

Methyl 9-fluoro-9H-fluorene-2-carboxylate was obtained by allowing methyl 9-oxo-9H-fluorene-2-carboxylate to react with sodium borohydride at room temperature in methanol to reduce the carbonyl group, and then allowing the thus obtained compound to react with diethylaminosulfur trifluoride at room temperature in methylene chloride.
FAB-MS: 243 (M + H)⁺.

### Reference Preparation 10

Propyl spiro[1,3-dioxolane-2,9'-fluorene]-2'-carboxylate was obtained by allowing propyl 9-oxo-9H-fluorene-2-carboxylate to react with ethylene glycol and p-toluenesulfonic acid under heating in benzene. FAB-MS: 311 (M + H)⁺.

### Reference Preparation 11

Propyl 9,9-dimethoxy-9H-fluorene-2-carboxylate was obtained by allowing propyl 9-oxo-9H-fluorene-2-carboxylate to react with methyl orthoformate and acetyl chloride in methanol at room temperature. FAB-MS: 313 (M + H)⁺.

### Reference Preparation 12

5'-Fluorospiro[1,3-dithiolane-2,9'-fluorene]-2'-carboxylate was obtained by allowing 5-fluoro-9-oxo-9H-fluorene-2-carboxylate to react with 1,2-ethanedithiol and boron trifluoride diethyl ether complex under heating in acetic acid. ESI-MS: 317 (M - H)⁻.

### Reference Preparation 13-a

Methyl (9EZ)-9-hydroxyimino-9H-fluoreue-2-carboxylate was obtained by allowing methyl 9-oxo-9H-fluorene-2-carboxylate to react with hydroxylamine hydrochloride at room temperature in pyridine. FAB-MS: 254 (M + H)⁺.

### Reference Preparation 13-b

Methyl 9-acetylamino-9H-fluorene-2-carboxylate was obtained by treating methyl (9EZ)-9-hydroxyimino-9H-fluorene-2-carboxylate with 10% palladium-carbon and acetic anhydride in dioxane in an atmosphere of hydrogen gas. FAB-MS: 282 (M + H)⁺.

### Reference Preparation 14-a

1-Biphenyl-2-ylcyclopentanol was obtained by allowing 2-bromobiphenyl to react with n-butyl lithium (1.58 M, hexane solution) at -78°C in THF, and then adding THF solution of cyclopentanone and carrying out the reaction at room temperature. EI-MS: 238 (M)⁺.

### Reference Preparation 14-b

Spiro[cyclopentane-1,9'-fluorene] was obtained by allowing 1-biphenyl-2-ylcyclopentanol to undergo the reaction under heating in formic acid. EI-MS: 220 (M)⁺.

### Reference Preparation 14-c

2'-Bromospiro[cyclopentane-1,9'-fluorene] was obtained by bromination described in J. Am. Chem. Soc., 80,4327 (1958) using spiro[cyclopentane-1,9'-fluorene].
EI-MS: 298 (M)⁺, 300 (M + 2)⁺.

### Reference Preparation 14-d

Spiro[cyclopentane-1,9'-fluorene]-2'-carbonitrile was obtained by allowing 2'-bromospiro[cyclopeirtane-1,9'-fluorene] to react with cupper cyanide under heating in DMF. ESI-MS: 246 (M + H)⁺.

### Reference Preparation 14-e

Spiro[cyclopentane-1,9'-fluorene]-2'-carboxylic acid was obtained by allowing ethanol solution of spiro[cyclopentane-1,9'-fluorene]-carbonitrile to react with 8 M potassium hydroxide aqueous solution under heating. ESI-MS: 263 (M + H)⁺.

### Reference Preparation 15

4-(4'-Methylbiphenyl2-yl)tetrahydro-2H-pyran-4-ol [EI-MS: 268 (M)⁺] was obtained by carrying out the reaction of Reference Preparation 14-a using 2-bromo-4'-methylbiphenyl and tetrahydro-4H-pyran-4-one as the starting materials, and then 2-methyl-2',3',5',6'-tetrahydrospiro[fluorene-9,4'-pyran] [ESI-MS: 251 (M + H)⁺] was produced in the same manner as in Reference Preparation 14-b. By allowing this to react with potassium permanganate under heating in pyridine-water, and allowing the thus obtained crude product to undergo the reaction under heating in methanol in the presence of sulfuric acid, methyl-2',3',5',6'-tetrahydrospiro[fluorene-9,4'-pyran]-2'-carboxylate was obtained. ESI-MS: 295 (M + H)⁺.

### Reference Preparation 16-a

9H-Fluorene-9,9-diyldimethylene dimethanesulfonate was obtained by allowing 9H-fluorene-9,9-diyldimethanol to react with methanesulfonyl chloride and triethylamine at room temperature in methylene chloride. EI-MS: 382 (M)⁺.

### Reference Preparation 16-b

9,9,-Bis(iodomethyl)-9H-fluorene was obtained by allowing 9H-fluorene-9,9-diyldimethylene dimethanesulfonate to react with sodium iodide under heating in hexamethylphosphoramide. By treating this with zinc under heating in ethanol, spiro[cyclopropane-1,9'-fluorene] was obtained (EI-MS: 192 (M)⁺). Thereafter, spiro[cyclopropane-1,9'-fluorene]-2'-carboxylic acid was produced in the same manner as in Reference Preparations 14-c to 14-e. ESI-MS: 235 (M - H)⁺.

### Reference Preparation 17-a

9,9-Bis[2-(benzyloxy)ethyl]-2-bromo-9H-fluorene was obtained by allowing 2-bromo-9H-fluorene and [(2-chloroethoxy)methyl]benzene to undergo the reaction under heating in DMSO in the presence of potassium tert-butoxide. FAB-MS: 535 (M + Na)⁺, 537 (M + 2 + Na)⁺.

### Reference Preparation 17-b

Ethyl 9,9-bis[2-(benzyloxy)ethyl]-9H-fluorene-2-carboxylate was obtained by cyanation of the bromo group of 9,9-bis[2-(benzyloxy)ethyl]-2-bromo-9H-fluorene in the same manner as in Reference Preparation 14-d and subsequently converting into carboxyl group by the same hydrolysis reaction of Reference Preparation 14-e, and then carrying out esterification reaction in the same manner as in Reference Preparation 4-b using ethyl iodide. FAB-MS: 507 (M+H)⁺.

### Reference Preparation 17-c

Ethyl 9,9-bis(2-hydroxyethyl)-9H-fluorene-2-carboxylate was obtained by allowing ethyl 9,9-bis[2-(benzyloxy)ethyl]-9H-fluoxene-2-carboxylate to react with palladium-carbon at room temperature in methanol in an atmosphere of hydrogen gas (FAB-MS: 327 (M + H)⁺). The thus obtained compound was allowed to react with p-toluenesulfonyl chloride at room temperature in methylene chloride in the presence of triethylamine, and then the thus obtained compound was allowed to react with methylamine (40% methanol solution) under heating in dioxane in the presence of potassium carbonate to obtain methyl 1'-methylspiro[fluorene-9,4'-piperidine]-2-carboxylate. APCI: 308 (M + H)⁺.

### Reference Preparation 18-a

Methyl 9,9-bis(hydroxymethyl)-9H-fluorene-2-carboxylate was obtained by allowing methyl 9H-fluorene-2-carboxylate to react with paraformaldehyde at room temperature in DMSO in the presence of sodium ethoxide. FAB-MS: 284 (M)⁺.

### Reference Preparation 18-b

Methyl 9,9-bis({[tert-butyl(dimethyl)silyl]oxy} methyl)-9H-fluorene-2-carboxylate was obtained by allowing methyl 9,9-bis(hydroxymethyl)-9H-fluorene-2-carboxylate to react with tert-butyldimethylsilyl chloride at room temperature in pyridine.
FAB-MS: 513 (M + H)⁺.

### Reference Preparation 19

9-Hydroxy-9-(tetrahydro-2H-pyran-4-yl)-9H-fluorene-2-carboxylic acid [FAB-MS: 309 (M - H)⁻] produced from 4-tetrahydro-2H-pyranyl magnesium chloride (prepared from 4-chlorotetrahydro-2H-pyran and magnesium) and 9-oxo-9H-fluorene-2-carboxylic acid in the same manner as in Reference Preparation 4-a was allowed to react with triethylsilane at room temperature in trifluoroacetic acid, thereby obtaining 9-(tetrahydro-2H-pyran-4-yl)-9H-fluorene-2-carboxylic acid. FAB-MS: 291 (M - H)⁻.

### Reference Preparation 20

9-(Tetrahydro-4H-pyran-4-ylidene)-9H-fluorene-2-carboxylic acid was obtained by allowing 9-hydroxy-9-(tehahydro-2H-pyran-4-y1)-9H-fluorene-2-carboxylic acid to react with 6 M hydrochloric acid under heating in dioxane. FAB-MS: 294 (M)⁺.

### Reference Preparation 21-a

2-Fluoro-4'-methyl-6-nitrobiphenyl [FAB-MS: 232 (M + H)⁺] was obtained from 2-fluoro-6-nitrophenyl trifluoromethanesulfonate and 4-methylphenylboric acid by carrying out the reaction in the same manner as in Reference Preparation 1-a, and converted into (6-fluoro-4'-methylbiphenyl-2-yl)amine [EI-MS: 201 (M)⁺] by subjecting this nitro group to catalytic hydrogenation reduction, and then Sandmeyer reaction was carried out to obtain 2-bromo-6-fluoro-4'-methylbiphenyl. EI-MS: 266 (M)⁺, 268 (M)⁺.

### Reference Preparation 21-b

4-(6-Fluoro-4'-methylbiphenyl-2-yl)tetrahydro-2H-pyran-4-ol [EI-MS: 286 (M)⁺] was obtained by carrying out the reaction of 2-bromo-6-fluoro-4'-methytbiphenyl with tetrahydro-4H-pyran-4-one in the same manner as in Reference Preparation 14-a using t-butyl lithium (1.48 M, pentane solution), and then further reaction was carried out in the same manner as in Reference Preparation 14-b to obtain 5-fluoro-2-methyl-2',3',5',6'-tetrahydrospiro[fluorene-9,4'-pyran]. FAB-MS: 268 (M)⁺.

### Reference Preparation 21-c

5-Fluoro-2',3',5',6'-tetrahydrospiro[fluarene-9,4'-pyran]-2-carboaldehyde [FAB-MS: 283 (M + H)⁺] was obtained by allowing 5-fluoro-2-methyl-2',3',5',6'-tetrahydrospiro[fluorene-9,4'-pyran], N-bromosuccinimide and 2,2'-azobisisobutyronitrile to undergo the reaction under heating in carbon tetrachloride, and by allowing the thus obtained crude product to react with silver nitrate in acetone-water. By further allowing this compound to react with sodium perchlorate, sodium dihydrogenphosphate and 2-methyl-2-butene at room temperature in a mixed solvent of tert-butanol-acetonitrile-water, 5-fluoro-2',3',5',6'-tetrahydrospiro[fluorene-9,4'-pyran]-2-carboxylic acid was obtained.
FAB-MS: 299 (M + H)⁺.

Compounds of Reference Preparations 22 to 111 were produced in the same manner as in the above Reference Preparations. Their structural formulae and physical properties are shown in the Tables 1 to 6 which are described later.

### Preparation 1

A 402 mg portion of CDI was added to 20 ml DMF solution of 400 mg 5-fluoro-9-oxo-9H-fluorene-2-carboxylic acid and stirred at 50°C for 1 hour. After cooling to room temperature, 743 mg of guanidine carbonate was added thereto and stirred overnight After evaporation of the solvent, water was added thereto, and the thus precipitated solid was purified by a silica gel column chromatography (Chromatorex (registered trademark), methanol/chloroform) to obtain 434 mg of N-(diaminomethylene)-5-fluoro-9-oxo-9H-fluorene-2-carboxamide as yellow solid.

### Preparation 2

A 2.67 g portion of 1,1'-carbonyldiimidazole was added to 60 ml dimethylformamide (DMF) solution of 3.35 g 9-oxo-9H-fluorene,-2-carboxylic acid and stirred at room temperature for 2.25 hours. This solution was added under ice-cooling to a solution which had been prepared by adding 3.00 g of sodium hydride to 20 ml DMF solution of 7.16 g guanidine hydrochloride and stirring at room temperature for 1.5 hours, and the mixture was stirred at room temperature for 1.5 hours. After evaporation of the solvent, water and ethyl acetate were added thereto, and the precipitated solid was washed with methanol to obtain 3.00 g of N-(diaminomethylene)-9-oxo-9H-fluorene-2-carboxamide as yellow solid.

### Preparation 3

A 110 mg portion of sodium borohydride was added to 10 ml methanol solution of 400 mg N-(diaminomethylene)-9-oxo-9H-fluorene-2-carboxamide and stirred at room temperature for I hour. After evaporation of the solvent, chloroform and 1 M sodium hydroxide aqueous solution were added thereto, and the precipitated solid was dissolved in 30 ml of ethanol, mixed with 0.2 ml of 4 M hydrogen chloride-ethyl acetate solution and stirred at room temperature for 1.5 hours. By collecting the thus formed solid by filtration, 380 mg of N-(diaminomethylene)-9-hydroxy-9H-fluorene-2-carboxamide hydrochloride was obtained as white solid.

### Preparation 4

A 1.0 g portion of sulfonyl chloride was added to 20 ml methylene chloride suspension of 480 mg N-(diaminomethylene)-9-hydroxy-9H-fluorene-2-carboxamide and stirred at room temperature for 30 minutes. After evaporation of the solvent, the residue was purified by a column chromatography (silica gel 60, methanol/chloroform) to obtain 155 mg of 9-chloro-N-(diaminomethylene)-9H-fluorene-2-carboxamide.

### Preparation 5

A 2 ml portion of 4 M hydrogen chloride-ethyl acetate solution was added to 10 ml methanol solution of 170 mg of tert-butyl (2-{[(diaminomethylene)amino]carbonyl}-9H-fluoren-9-yl)carbamate produced in the same manner as in Preparation 1, and the mixture was stirred at 60°C for 20 minutes. By washing the thus obtained solid with hot ethanol, 83 mg of 9-amino-N-(diaminomethylene)-9H-fluorene-2-carboxamide dihydrochloride was obtained.

### Preparation 6

A 1 ml portion of 4 M hydrogen chloride-methanol solution was added to 5 ml methanol solution of 490 mg N-(diaminomethylene)-9,9-bis({[tert-butyl(dimethyl)silyl]oxy}methyl)-9H-fluorene-2-carboxamide and stirred at room temperature for 2 hours. By evaporating the solvent and washing the residue with ethyl acetate, 250 mg of N-(diaminomethylene)-9,9-bis(hydroxymethyl)-9H-fluorene-2-carboxamide hydrochloride was obtained.

### Preparation 7

A 20 mg portion of hydroxylamine hydrochloride was added to 3 ml pyridine solution of 55 mg N-(diaminomethylene)-8-hydroxymethyl-9-oxo-9H-fluorene-2-carboxamide and stirred at room temperature for 10 hours. After evaporation of the solvent, water-ethanol was added thereto, and the precipitated solid was purified by Chromatorex (methanol/chloroform) to obtain 14 mg of (9EZ)-N-(diaminomethylene)-9-hydroxyimino-8-hydroxymethyl-9H-fluorene-2-carboxamide as white solid.

### Preparation 8

A 420 mg portion of (2R)-2-[(tert-butoxycarbonyl)amino]-3-methylbutanoic acid, 400 mg of 1-[3-(dimethylamino)propyl]-3-ethylcarbodiimide and 22 mg of 4-(N,N-dimethylamino)pyridine were added to 8 ml DMF solution of 470 mg N-(diaminomethylene)-9-hydroxy-9H-fluorene-2-carboxamide and stirred overnight at room temperature. After evaporation of the solvent, chloroform and saturated sodium bicarbonate aqueous solution were added thereto to remove the insoluble matter, and the organic layer was washed with saturated brine and dried with magnesium sulfate. After evaporation of the solvent, the residue was purified by a column chromatography (silica gel 60, methanol/chloroform). A 270 mg portion of the thus obtained compound was dissolved in 10 ml of ethanol, mixed with 2 ml of 4 M hydrogen chloride-ethyl acetate solution and stirred overnight at 40°C. After evaporation of the solvent, the residue was recrystallized from 2-propanol to obtain 30 mg of 9-hydroxy-N-[(2EZ,4S)-4-isopropyl-5-oxoimidazolin-2-ylidene-9H-fluorene-2-carboxamide hydrochloride.

### Preparation 9

A 2.99 ml portion of sodium methoxide-methanol solution (28%) was added to 8 ml DMF solution of 1.38 g guanidine hydrochloride and stirred at room temperature for 1 hour. A 4 ml portion of DMF solution of 350 mg ethyl 6-methyl-9-oxo-9H-fluorene-2-carboxylate was added to this solution and stirred at 100°C for 3 hours. This was spontaneously cooled to room temperature, the solvent was evaporated, and then methanol, water and 1 M sodium hydroxide aqueous solution were added thereto, and the thus precipitated solid was washed with 5 ml of methanol to obtain 215 mg of N-(diaminomethylene)-6-methyl-9-oxo-9H-fluorene-2-carboxamide as yellow solid.

Compounds of Preparations 10 to 110 were produced in the same manner as in the above Preparations. Their structural formulae and physical properties are shown in the Tables 7 to 18 which are described later. In addition, the compounds of Table 19 and Table 20, which are described later, may be easily produced in almost the same manner as in the above Preparations or the methods described in the production methods, or by applying slight modifications obvious for those skilled in the art to these methods.

In this connection, the 6 compounds of Preparations 56a and 56b, 60a and 60b, 78a and 78b were produced by carrying out resolution of respective compounds of Preparations 56, 60 and 77 produced as racemic compounds, using chiral columns. The used columns and solvents used as the mobile phase are shown below.

### Preparations 56a and 56b

Used column: CHIRALPAK AD-H, mobile phase: medmol/diethylarnine.

### Preparations 60a and 60b

Used column: CHIRALPAK OJ, mobile phase: ethanol/diethylamine.

### Preparations 78a and 78b

Used column: CHIRALPAK AD-H, mobile phase: hexane/ethanol/triethylamine.

### Preparations 111

2-amino-4-(4-fluoronaphtho-1-yl)-6-isopropylpyrimidine(hereinafter abbreviated as RS-127445) was prepared in the same manner as described in the international application WO97/44326, and (R)-3-(2-(4-methylpiperidine-1-yl)ethyl)pyrrolidine-1-sulfonyl)phenol, (hereinafter abbreviated as SB-269970) was prepared in the same manner as described in the international application WO97/48681, respectively.

Symbols in the tables have the following meanings. Each numeral before the substituent group shows the substituted position.

Me, methyl, Et: ethyl, Bu: normal butyl, REx: Reference Preparation number, Ex: Preparation number, Cmp: compound number, RSyn and Syn: production methods (numerals indicate Reference Preparation number and Preparation number of compound produced in the same manner), Str: structure, Sal: salt (not described: free form; HCl: hydrochloride; numeral indicates molar ratio of the acid component, for example, 2 HCl means dihydrochloride), Dat: physicochemical properties(FAB: FAB-MS, ESI: ESI-MS, EI: EI-MS, NMR: 8 values of typical peaks of nuclear magnetic resonance spectrum (DMSO-_{d6}, TMS internal standard)).

In this connection, the compound in which "*" was added to the substituent group in respective table indicates that it is one of the chiral compounds obtained by separation of optical isomers based on the asymmetry of carbon bonded to said substituent group. In addition, the following symbols shown in the tables show analytical conditions of high performance liquid chromatography. Proc. A: (column: CHIRALPAK AD-H [0.46 cm I.D. x 25 cm], mobile phase, methanol/diethylamine = 100/0.1, flow rate: 0.5 ml/min, temperature: 20°C, wavelength: 260 nM), Proc. B: (column: CHIRALPAK OJ [0.46 cm I.D. x 25 cm], mobile phase, ethanol/diethylamine = 100/0.1, flow rate: 0.3 ml/min, temperature: 40°C, wavelength: 264 nM), Proc. C: (column: CHIRALPAK AD-H [0.46 cm I.D. x 25 cm], mobile phase, hexane/ethanol/triethylamine = 50/50/0.05, flow rate: 1.0 ml/min, temperature: 25°C, wavelength: 257 nM).

**Table 1**

| | | | | |
|---|---|---|---|---|
| 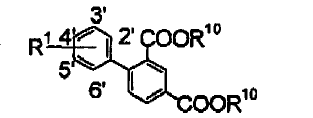 | | | | |

| REx | RSyn | R¹ | R¹⁰ | Dat |
|---|---|---|---|---|
| 22 | 1-a | 2'-F | -Et | ESI : 317 (M+H)⁺ |
| 23 | 1-a | 3'-F | -Et | FAB : 317 (M+H)⁺ |
| 24 | 1-a | 4'-F | -Et | FAB : 317 (M+H)⁺ |
| 25 | 1-a | 2'-Cl | -Et | FAB : 333 (M+H)⁺ |
| 26 | 1-a | 3'-Cl | -Et | FAB : 333 (M+H)⁺ |
| 27 | 1-a | 4'-Cl | -Et | FAB : 333 (M+H)⁺ |
| 28 | 1-a | 3'-Me | -Et | FAB : 313 (M+H)⁺ |
| 29 | 1-a | 3'-OMe | -Et | FAB : 328 (M)⁺ |
| 30 | 1-a | 4'-OMe | -Et | FAB : 329 (M+H)⁺ |
| 31 | 1-a | 2'-Et | -Et | ESI : 327 (M+H)⁺ |
| 32 | 1-a | 2'-CF₃ | -Et | FAB : 367 (M+H)⁺ |
| 33 | 1-a | 2'-F, 5'-Me | -Et | ESI : 331 (M+H)⁺ |
| 34 | 1-a | 2'-Me, 5'-Me | -Et | ESI : 327 (M+H)⁺ |
| 35 | 1-b | 2'-F | -H | FAB : 261 (M+H)⁺ |
| 36 | 1-b | 3'-F | -H | FAB : 259 (M-H)⁻ |
| 37 | 1-b | 4'-F | -H | FAB : 261 (M+H)⁺ |
| 38 | 1-b | 2'-Cl | -H | FAB : 277 (M+H)⁺ |
| 39 | 1-b | 3'-Cl | -H | FAB : 277 (M+H)⁺ |
| 40 | 1-b | 4'-Cl | -H | FAB : 277 (M+H)⁺ |
| 41 | 1-b | 3'-Me | -H | FAB: 257 (M+H)⁺ |
| 42 | 1-b | 3'-OMe | -H | FAB : 271 (M-H)⁻ |
| 43 | 1-b | 4'-OMe | -H | FAB : 273 (M+H)⁺ |
| 44 | 1-b | 2'-Et | -H | FAB : 269 (M-H)⁻ |
| 45 | 1-b | 2'-CF₃ | -H | FAB : 309 (M-H)⁻ |
| 46 | 1-b | 2'-F, 5'-Me | -H | ESI : 273 (M-H)⁻ |
| 47 | 1-b | 2'-Me, 5'-Me | -H | FAB: 269 (M-H)⁻ |

**Table 2**

| 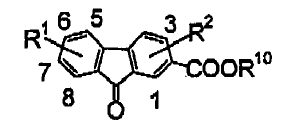 | | | | | |
|---|---|---|---|---|---|
| REx | RSyn | R¹ | R² | r¹⁰ | Dat |
| 48 | 1-c | 5-F | -H | -H | FAB:242(M)⁻ |
| 49 | 1-c | 7-F | -H | -H | FAB:243(M+H)⁺ |
| 50 | 1-c | 8-F | -H | -H | FAB:243(M+H)⁺ |
| 51 | 1-c | 5-Cl | -H | -H | FAB:259(M+H)⁺ |
| 52 | 1-c | 7-Cl | -H | -H | FAB:259(M+H)⁺ |
| 53 | 1-c | 8-Me | -H | -H | FAB:239(M+H)⁺ |
| 54 | 1-c | 5-Et | -H | -H | FAB:251(M-H)⁻ |
| 55 | 1-c | 5-CF₃ | -H | -H | FAB:291(M-H)⁻ |
| 56 | 1-c | 7-OMe | -H | -H | FAB:255(M+H)⁻ |
| 57 | 1-c | 5-F, 8-Me | -H | -H | ESI: 255 (M-H)⁻ |
| 58 | 1-c | 5-Me, 8-Me | -H | -H | FAB : 251 (M-H)⁻ |
| 59 | 1-c | -H | 1-Cl | -H | FAB : 259 (M+H)⁺ |
| 60 | 1-c | -H | 3-Cl | -H | FAB : 259 (M+H)⁺ |
| 61 | 2 | 5-F | -H | -Et | FAB : 271 (M+H)⁺ |
| 62 | 2 | 8-F | -H | -Et | FAB : 271 (M+H)⁺ |
| 63 | 2 | 5-Me | -H | -Me | FAB : 253 (M+H)⁺ |
| 64 | 2 | 6-OMe | -H | -Et | FAB : 283 (M+H)⁺ |
| 65 | 2 | 8-OMe | -H | -Et | FAB : 283 (M+H)⁺ |
| 66 | 2 | -H | -Et | -Et | FAB : 287 (M+H)⁺ |
| 67 | 2 | -H | -Et | -Et | FAB : 287 (M+H)⁺ |
| 68 | 4-b | 6-Cl | -H | -CH₂CH=CH₂ | FAB : 299 (M+H)⁺ |
| 69 | 4-b | 8-Cl | -H | -CH₂CH=CH₂ | FAB : 299 (M+H)⁺ |
| 70 | 7-b | 5-CH₂NMe₂ | -H | -Me | FAB : 296 (M+H)⁺ |
| 71 | 8-a | 5-CH₂OAc | -H | -Me | FAB : 311 (M+H)⁺ |
| 72 | 8-c | 5-CH₂OMe | -H | -Me | FAB : 283 (M+H)⁺ |

**Table 3**

| | | | | | | |
|---|---|---|---|---|---|---|
| 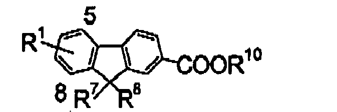 | | | | | | |

| REx | RSyn | R¹ | R⁷ | R⁸ | R¹⁰ | Dat |
|---|---|---|---|---|---|---|
| 73 | 4-a | -H | -Et | -OH | -H | FAB : 253 (M-H)⁻ |
| 74 | 4-a | -H | -Bu | -OH | -H | ESI : 281 (M-H)⁻ |
| 75 | 4-a | 5-F | -Me | -OH | -H | FAB : 257 (M-H)⁻ |
| 76 | 4-a | 5-Me | -Me | -OH | -H | EI : 254 (M)⁺ |
| 77 | 4-a | 5-Et | -Me | -OH | -H | FAB: 267 (M-H)⁻ |
| 78 | 4-a | 5-CF₃ | -Me | -OH | -H | FAB : 307 (M-H)⁻ |
| 79 | 4-a | 8-Me | -Me | -OH | -H | FAB : 253 (M-H)⁻ |
| 80 | 4-a | 5-F, 8-Me | -Me | -OH | -H | FAB : 271 (M-H)⁻ |
| 81 | 4-a | 5-Me, 8-Me | -Me | -OH | -H | FAB : 267 (M-H)⁻ |
| 82 | 4-b | -H | -Et | -OH | -Me | FAB : 269 (M+H)⁺ |
| 83 | 4-b | 5-F | -Me | -OH | -Me | EI : 272 (M)⁺ |
| 84 | 4-b | 8-Me | -Me | -OH | -Me | EI : 268 (M)⁺ |
| 85 | 4-b | 5-F, 8-Me | -Me | -OH | -Me | FAB : 285 (M-H)⁻ |
| 86 | 4-c | -H | -Et | -OMe | -Me | FAB : 283 (M+H)⁺ |
| 87 | 4-c | 5-F | -Me | -OMe | -Me | FAB : 287 (M+H)⁺ |
| 88 | 4-c | 8-Me | -Me | -OMe | -Me | FAB : 283 (M+H)⁺ |
| 89 | 4-c | 5-F, 8-Me | -Me | -OMe | -Me | FAB: 301 (M+H)⁺ |
| 90 | 5 | -H | -Me | -O(CH₂)₂OMe | -Me | EI : 312 (M)⁺ |
| 91 | 7-b | 5-F, 8-CH₂-NMe₂ | -Me | -OMe | -Me | FAB : 344 (M+H)⁺ |
| 92 | 7-b | 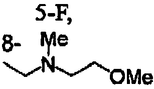 | -Me | -OMe | -Me | FAB : 388 (M+H)⁺ |
| 93 | 14-e | -H | -CH₂OMe | -CH₂OMe | -H | ESI : 297 (M-H)⁻ |
| 94 | 14-e | -H | -(CH₂)₂OMe | -(CH₂)₂OMe | -H | BSI : 325 (M-H)⁻ |
| 95 | 14-e | -H | -(CH₂)₂OBn | -(CH₂)₂OBn | -H | ESI : 477 (M-H)⁻ |
| 96 | 1-b | -H | -F | -H | -H | FAB: 227 (M-H)⁻ |

**Table 4**

| | | | | | | |
|---|---|---|---|---|---|---|
| 97 | 1-b | -H | -NHAc | -H | -H | FAB : 268 (M+H)⁺ |
| 98 | 1-b | -H | -NHBOC | -H | -H | FAB : 338 (M-H)⁻ |
| 99 | 1-b | -H | -(CH₂)₃OH | -OH | -H | EI : 284 (M)⁺ |
| 100 | 1-b | -H | -CH₂OTBS | -CH₂OTBS | -H | FAB : 499 (M+H)⁺ |

**Table 5**

| | | | | | |
|---|---|---|---|---|---|
| 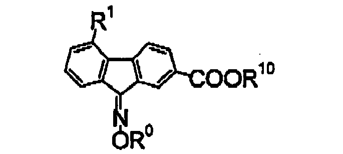 | | | | | |

| REx | RSyn | R⁰ | R¹ | R¹⁰ | Dat |
|---|---|---|---|---|---|
| 101 | 13-a | -H | -F | -H | FAB; 286 (M+H)⁺ |
| 102 | 13-a | -Me | -H | -Pr | FAB : 296 (M+H)⁺ |

**Table 6**

| | | | | | |
|---|---|---|---|---|---|
| 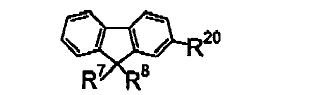 | | | | | |

| REx | RSyn | R⁷ | R⁸ | R²⁰ | Dat |
|---|---|---|---|---|---|
| 103 | 5 | -CH₂OMe | -CH₂OMe | -H | ESI : 255 (M+H)⁺ |
| 104 | 14-b | -(CH₂)₂O(CH₂)₂- | | -Me | FAB: 251 (M+H)⁺ |
| 105 | 14-c | -CH₂OMe | -CH₂OMe | -Br | ESI : 332(M+H)⁺, 334 (M+H+2)⁺ |
| 106 | 14-c | -(CH₂)₂OMe | -(CH₂)₂OMe | -Br | FAB : 361(M+H)⁺, 363 (M+H+2)⁺ |
| 107 | 14-c | -(CH₂)₂- | | -Br | EI : 270(M)⁺ 272 (M+2)⁺ |
| 108 | 14-d | -CH₂OMe | -CH₂OMe | -CN | ESI : 280 (M+H)⁺ |
| 109 | 14-d | -(CH₂)₂OMe | -(CH₂)₂OMe | -CN | ESI : 308 (M+H)⁺ |
| 110 | 14-d | -(CH₂)₂- | | -CN | FAB : 218 (M+H)⁺ |
| 111 | 17-a | -(CH₂)₂OMe | -(CH₂)₂OMe | -H | FAB : 283 (M+H)⁺ |

**Table 7**

| | | | |
|---|---|---|---|
| 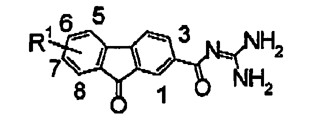 | | | |

| Ex | Syn | R¹ | Dat |
|---|---|---|---|
| 1 | 1 | 5-F | NMR: 7.45-7.55 (3H, m), 7.73 (1H, d, *J*= 7.3 Hz), 8.36 (1H, s). ; FAB : 284 (M+H)⁺ |
| 2 | 2 | -H | NMR : 7.42 (1H, t, *J*= 8.3 Hz), 7.62-7.67 (2H, m), 8.31 (1H, s). ; FAB : 266 (M+H)⁺ |
| 9 | 9 | 6-Me | NMR: 2.42 (3H, s), 7.22 (1H, d, *J* = 7.3 Hz), 8.29 (1H, s). ; FAB : 280 (M+H)⁺ |
| 10 | 1 | 7-F | NMR: 7.46-7.51 (2H, m), 8.28 (1H, dd, *J* = 7.8, 1.5 Hz), 8.31 (1H, d, *J*=1.2 Hz). ; FAB : 284 (M+H)⁺ |
| 11 | 1 | 8-F | NMR: 7.21 (1H, ddd, *J* = 9.8, 8.3, 2.5 Hz), 7.70 (1H, dd, J= 8.3, 5.3 Hz), 8.31 (1H, s). ; FAB : 284 (M+H)⁺ |
| 12 | 2 | 1-Cl | NMR : 7.43 (1H, t, *J* = 6.8 Hz), 7.62-7.69 (3H, m), 7.75(1H, d, *J* = 7.8 Hz). ; FAB: 300 (M+H)⁺ |
| 13 | 2 | 3-Cl | NMR : 7.44 (1H, t, *J* = 7.3 Hz), 7.79 (1H, s), 7.94(1H, s). ; FAB : 300 (M+H)⁺ |
| 14 | 2 | 5-Cl | NMR: 7.44 (1H, t, *J* = 7.8 Hz), 8.18 (1H, d, *J* = 7.8 Hz), 8.37 (1H, d, *J* = 1.0 Hz). ; FAB : 300 (M+H)⁺ |
| 15 | 2 | 7-Cl | NMR: 7.64 (1H, d, *J* = 1.9 Hz), 7.84-7.89 (2H, m), 8.32 (1H, s). ; FAB : 300 (M+H)⁺ |
| 16 | 2 | 5-Me | NMR : 2.61 (3H, s), 7.31 (1H, t, *J* = 7.6 Hz), 8.33 (1H, d, *J* = 0.9 Hz). ; FAB : 280 (M+H)⁺ |
| 17 | 2 | 5-Et | NMR : 1.29 (3H, t, *J* = 7.3 Hz), 7.36 (1H, t, *J=* 7.3 Hz), 8.33 (1H, d, *J=* 1.4 Hz). ; FAB : 294 (M+H)⁺ |
| 18 | 2 | 7-OMe | NMR : 3.85 (3H, s), 7.69 (1H, d, *J=* 7.8 Hz), 8.26 (1H, s). ; FAB : 296 (M+H)⁺ |
| 19 | 9 | 6-Cl | NMR : 7.64 (1H, d, *J* = 7.3 Hz), 8.02 (1H, d, *J* = 1.5 Hz), 8.33(1H, s). ; FAB : 300 (M+H)⁺ |
| 20 | 9 | 8-Cl | NMR : 7.40 (1H, *d, J=* 7.8 Hz), 7.63 (1H, t, *J* = 7.8 Hz), 8.32(1H, s). ; FAB : 300 (M+H)⁺ |
| 21 | 9 | 8-Me | NMR : 2.56 (3H, s), 7.19 (1H, d, *J* = 7.8 Hz), 8.27 (1H, s). ; FAB : 280 (M+H)⁺ |
| 22 | 9 | 6-OMe | NMR : 3.92 (3H, s), 6.90 (1H, dd, *J* = 8.3 Hz, 2.0 Hz), 8.26-8.27 (2H, m). ; FAB : 296 (M+H)⁺ |
| 23 | 9 | 8-OMe | NMR : 3.91 (3H, s), 7.61 (1H, t, *J* = 8.1 Hz), 8.26 (1H, s). ; FAB : 296 (M+H)⁺ |
| 24 | 9 | 5-CH₂NMe₂ | NMR : 2.45 (6H, s), 3.66 (2H, s), 8.32 (1H, d, *J* = 1.0 Hz). ; FAB : 323 (M+H)⁺ |

**Table 8**

| | | | |
|---|---|---|---|
| 25 | 9 | 8-CH₂NMe₂ | NMR : 2.21 (6H, s), 3.85 (2H, s), 8.25 (1H, d, *J* = 1.4 Hz). ; FAB : 323 (M+H)⁺ |
| 26 | 9 | 5-CH₂OH | NMR : 4.84 (2H, d, *J* = 4.4 Hz), 7.41 (1H, t, *J* = 7.4 Hz), 8.32 (1H, d, *J* = 1.4 Hz). ; FAB : 296 (M+H)⁺ |
| 27 | 9 | 8-CH₂OH | NMR : 4.94 (2H, d, *J* = 5.9 Hz), 7.55 (1H, dd, *J* = 7.8, 1.0 Hz), 8.25 (1H, s). ; FAB : 295 (M)⁺ |
| 28 | 9 | 5-CH₂OMe | NMR : 3.42 (3H, s), 4.75 (2H, s), 8.34 (1H, d, *J* = 1.4 Hz). ; FAB : 310 (M+H)⁺ |
| 29 | 9 | 8-CH₂OMe | NMR : 3.40 (3H, s), 4.86 (2H, s), 8.26 (1H, s). ; FAB: 310 (M+H)⁺ |

**Table 9**

| | | | | |
|---|---|---|---|---|
| 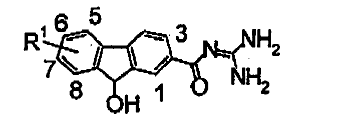 | | | | |

| Ex | Syn | R¹ | Sal | Dat |
|---|---|---|---|---|
| 3 | 3 | -H | HCl | NMR: 5.59 (1H, s), 7.40-7.49 (2H, m), 8.23-8.28 (2H, m). ; FAB : 268 (M+H)⁺ |
| 30 | 3 | 5-F | HCl | NMR: 5.67 (1H, s), 7.30 (1H, t, *J* = 8.3 Hz), 8.28 (1H, s). ; FAB : 286 (M+H)⁺ |
| 31 | 3 | 7-F | | NMR : 5.49 (1H, d, *J* = 7.3 Hz), 7.18-7.26 (1H, m), 8.32 (1H, s). ; FAB : 286 (M+H)⁺ |
| 32 | 3 | 8-F | | NMR : 5.48 (1H, d, J= 7.8 Hz), 7.14 (1H, ddd, *J* = 9.8, 8.3, 2.4 Hz), 8.33 (1H, s). ; FAB : 286 (M+H)⁺ |
| 33 | 3 | 1-Cl | HCl | NMR : 5.67 (1H, s), 7.43-7.49 (2H, m); 7.65 (1H, dd, *J* = 6.3, 2.0 Hz). ; FAB : 302 (M+H)⁺ |
| 34 | 3 | 3-Cl | HCl | NMR : 5.56 (1H, s), 7.42-7.48 (2H, m), 8.13 (1H, s). ; FAB : 302 (M+H)⁺ |
| 35 | 3 | 5-Cl | HCl | NMR : 5.63 (1H, s), 7.65 (1H,d, *J=* 6.8 Hz), 8.29-8.31 (2H, m). ; FAB : 302 (M+H)⁺ |
| 36 | 3 | 6-Cl | HCl | NMR :5.59 (1H, s), 7.47 (1H, dd, *J=* 8.1, 1.7 Hz), 8.08-9.10 (2H, m),. ; FAB : 302 (M+H)⁺ |
| 37 | 3 | 7-Cl | HCl | NMR : 5.59 (1H, d, *J* = 7.3 Hz), 7.65 (1H,s), 8.27 (1H, s). ; FAB : 302 (M+H)⁺ |
| 38 | 3 | 8-Cl | HCl | NMR : 5.70 (1H, s), 7.49 (1H, t, *J* = 7.8 Hz), 8.25 (1H, s). ; FAB : 302 (M+H) ⁺ |
| 39 | 3 | 5-Me | HCl | NMR : 2.67 (3H, s), 5.54 (1H, s), 8.27 (1H, s). ; FAB : 282 (M+H)⁺ |

**Table 10**

| | | | | |
|---|---|---|---|---|
| 40 | 3 | 6-Me | HCl | NMR : 2.41 (3H, s), 5.54 (1H, s), 8.22 (1H, s). ; FAB : 282 (M+H)⁺ |
| 41 | 3 | 8-Me | HCl | NMR : 2.50 (3H, s), 5.64 (1H, s), 8.20 (1H, s). ; FAB : 282 (M+H)⁺ |
| 42 | 3 | 5-Et | | NMR : 1.28 (3H, t, *J* = 7.3 Hz), 5.42 (1H, d, *J* = 7.3 Hz), 8.34 (1H, s). ; FAB : 296 (M+H)⁺ |
| 43 | 3 | 6-OMe | HCl | NMR: 3.85 (3H, s), 5.52 (1H, s), 8.21 (1H, s). ; FAB : 298 (M+H)⁺ |
| 44 | 3 | 7-OMe | HCl | NMR : 3.85 (3H, s), 5.53 (1H, s), 8.20 (1H, s). ; FAB : 298 (M+H)⁺ |
| 45 | 3 | 8-OMe | HCl | NMR :3.89 (3H, s), 5.66 (2H, brs), 8.20 (1H, s). ; FAB : 298 (M+H)⁺ |
| 46 | 3 | 5-CH₂NMe₂ | 2HCl | NMR : 2.85 (6H, s), 5.58 (1H, s), 8.32 (1H, s). ; FAB : 325 (M+H)⁺ |
| 47 | 3 | 8-CH₂NMe₂ | 2HCl | NMR : 2.79 and 2.85 (6H, s and s), 5.99 (1H, s), 8.33 (1H, s). ; FAB : 325 (M+H)⁺ |
| 48 | 3 | 5-CH₂OH | HCl | NMR : 4.89 (2H, s), 5.56 (1H, s), 8.24 (1H, s). ; FAB : 298 (M+H)⁺ |
| 49 | 3 | 8-CH₂OH | HCl | NMR : 4.84 (2H, s), 5.69 (1H, s), 7.44-7.50 (2H, m). ; FAB : 298 (M+H)⁺ |
| 50 | 3 | 5-CH₂OMe | HCl | NMR : 3.39 (3H, s), 5.57 (1H, s), 8.23 (1H, s). ; FAB : 312 (M+H)⁺ |
| 51 | 3 | 8-CH₂OMe | HCl | NMR : 3.39 (3H, s), 5.69 (1H, s), 8.24 (1H, s). ; FAB : 312 (M+H)⁺ |

**Table 11**

| | | | | | |
|---|---|---|---|---|---|
| 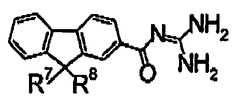 | | | | | |

| Ex | Syn | R⁷ | R⁸ | Sal | Dat |
|---|---|---|---|---|---|
| 4 | 4 | -H | -Cl | | NMR : 6.26 (1H, s), 7.40-7.45 (1H, m), 8.36 (1H, s). ; FAB : 286 (M+H)⁺ |
| 5 | 5 | -H | -NH₂ | 2HCl | NMR: 5.54 (1H, brs), 7.54 (1H, dt, *J*= 7.3, 1.0 Hz), 8.61 (1H, s). ; FAB : 267 (M+H)⁺ |
| 6 | 6 | -CH₂OH | -CH₂OH | HCl | NMR : .3.78 (4H, s), 7.38-7.46 (2H, m), 8.34 (1H, d, *J* = 0.9 Hz). ; FAB : 312 (M+H)⁺ |

**Table 12**

| | | | | | |
|---|---|---|---|---|---|
| 52 | 2 | -H | -H | | NMR: 3.96 (2H, s), 7.31-7.42 (2H, m), 8.31 (1H, s). ; FAB : 252 (M+H)⁺ |
| 53 | 2 | -H | -Me | HCl | NMR: 1.54 (3H, d, *J=* 7.3 Hz), 4.07 (1H, q, *J =* 7.3 Hz), 8.49 (1H, s). ; FAB : 266 (M+H)⁺ |
| 54 | 2 | -H | -F | HCl | NMR : 6.60 (1H, d, *J* = 52.7 Hz), 7.49 (1H, t, *J* = 7.8 Hz), 8.36 (1H, s). ; FAB : 270 (M+H)⁺ |
| 55 | 2 | -H | -NHCOCH₃ | HCl | NMR: 1.98 (3H, s), 6.11 (1H, d, *J=* 7.3 Hz), 8.14 (1H, s). ; FAB : 309 (M+H)⁺ |
| 56 | 2 | -OH | -Me | HCl | NMR: 1.64 (3H, s), 7.39-7.43 (2H, m), 8.26 (1H, d, *J =* 1.4 Hz). ; FAB : 282 (M+H)⁺ |
| 56a | | -OH* | -Me* | HCl | RT : 7.39, Proc.A ; FAB : 282 (M+H)⁺ |
| 56b | | -OH* | -Me* | HCl | RT : 11.98, Proc.A; FAB : 282 (M+H)⁺ |
| 57 | 1 | -OH | -Et | HCl | NMR : 0.42 (3H, t, *J* = 7.3 Hz), 2.04-2.18 (2H, m), 8.19 (1H, d, *J*= 1.0 Hz). ; FAB : 296 (M+H)⁺ |
| 58 | 2 | -OH | -Bu | HCl | NMR: 0.65-0.75 (5H, m), 7.39-7.44 (2H, m), 8.18 (1H, brs) ; FAB : 324 (M+H)⁺ |
| 59 | 2 | -S(CH₂)₂S- | | HCl | NMR : 3.83-3.90 (4H, m), 7.46-7.50 (2H, m), 8.57 (3H, m); FAB : 342 (M+H)⁺ |
| 60 | 9 | -O(CH₂)₃- | | HCl | NMR: 2.25-2.44 (4H, m), 7.39-7.46 (2H, m), 8.37 (1H, d, *J* = 1.5 Hz). ; FAB : 308 (M+H)⁺ |
| 60a | | -O(CH₂)₃-* | | HCl | RT : 14.34, Proc.B ; FAB : 308 (M+H)⁺ |
| 60b | | -O(CH₂)₃-* | | HCl | RT: 18.66, Proc.B ; FAB : 308 (M+H)⁺ |
| 61 | 1 | -CH₂OMe | -CH₂OMe | HCl | NMR: 3.17 (6H, s), 3.45 (4H, s), 8.39 (1H, d, *J*= 1.0 Hz).; FAB: 340 (M+H)⁺ |
| 62 | 1 | -(CH₂)₂OMe | -(CH₂)₂OMe | HCl 8.56 (1H, s). ; FAB : 368 (M+H)⁺ | NMR : 2.33-2.48 (4H, m), 2.87 (6H, s), |
| 63 | 1 | -(CH₂)₂- | | HCl | NMR : 1.81-1.86 (2H, m), 7.27-7.31 (1H, m), 8.20 (1H, d, *J =* 1.4 Hz). ; FAB: 278 (M+H)⁺ |

**Table 13**

| | | | | | |
|---|---|---|---|---|---|
| 64 | 1 | -(CH₂)₄- | | HCl | NMR : 1.94-2.02 (2H, m), 7.38-7.45 (2H, m), 8.40 (1H, s),. ; FAB : 306 (M+H)⁺ |
| 65 | 1 | -OH | 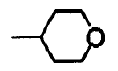 | HCl | NMR: 1.37 (2H, d, *J* = 1.7 Hz), 7.40-7.46 (2H, m), 8.12 (1H, s). ; FAB: 352 (M+H)⁺ |
| 66 | 1 | -H | 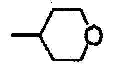 | HCl | NMR: 4.12 (1H, d, *J* = 3.0 Hz), 7.43-7.48 (2H, m), 8.30 (1H, s). ; FAB : 335 (M+H)⁺ |
| 67 | 1 | 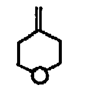 | | HCl | NMR : 3.21-3.24 (2H, m), 7.43-7.45 (2H, m), 8.57 (1H, s). ; FAB: 334 (M+H)⁺ |
| 68 | 9 | -OH | -OCH₂OMe | | NMR: 1.64 (3H, s), 3.04 (3H, s), 8.24 (1H, d, *J* = 1.5 Hz). ; FAB : 326 (M+H)⁺ |
| 69 | 9 | -OH | -O(CH₂)₂OMe | HCl | NMR : 1.67 (3H, s), 3,13 (3H, s), 8.22-8.24 (3H, m). ; FAB : 340 (M+H)⁺ |
| 70 | 9 | -OH | -O(CH₂)₃OH | HCl | NMR : 0.87-0.94 (2H, m), 3.19 (2H, t, *J* = 6.6 Hz), 8.17 (1H, s). ; FAB : 326(M+H)⁺ |
| 71 | 9 | -OMe | -Me | HCl | NMR : 1.67 (3H, s), 2.65 (3H, s), 8.30 (1H, d, *J* = 1.5 Hz).; FAB : 296 (M+H)⁺ |
| 72 | 9 | -OMe | -Et | HCl | NMR : 0.43 (3H, t, *J*= 7.6 Hz), 2.68 (3H, s), 8.17 (1H, d, *J* = 1.5 Hz).; FAB: 310(M+H)⁺ |
| 73 | 9 | -OMe | -OMe | | NMR: 3.29 (6H, s), 7.36 (1H, dt, *J*= 0.9, 7.6 Hz), 8.24 (1H, s).; FAB : 312 (M+H)⁺ |
| 74 | 9 | -O(CH₂)₂O- | | | NMR : 4.33-4.42 (4H, m), 7.32 (1H, dt, *J* = 1.0, 7.3 Hz), 8.21 (1H, s).; FAB: 310 (M+H)⁺ |
| 75 | 9 | -(CH₂)₂O(CH₂)₂- | | HCl | NMR : 1.76 (2H, dt, *J*= 13.7 Hz, 4.9 Hz), 4.06-4.11 (4H, m), 8.52 (1H, s). ; FAB : 322 (M+H)⁺ |
| 76 | 9 | -(CH₂)₂NMe(CH₂)₂- | | 2HCl | NMR: 1.63-1.71 (2H, m), 2.54 (3H, s), 8.92 (1H, s). ; ESI: 335 (M+H)⁺ |

**Table 14**

| 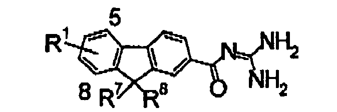 | | | | | | |
|---|---|---|---|---|---|---|
| Ex | Syn t | R¹ | R⁷ | R⁸ | Sal | Da |
| 77 | 1 | 5-F | -OH | -Me | HCl | NMR : 1.69 (3H, s), 7.25-7.30(1H, m), 8.29 (1H, d, *J* = 1.5 Hz). ; FAB : 300 (M+H)⁺ |
| 78a | | 5-F | -OH* | -Me* | HCl | RT : 3.97, Proc.C ;FAB : 300 (M+H)⁺ |
| 78b | | 5-F | -OH* | -Me* | HCl | RT : 5.78, Proc.C ; FAB : 300 (M+H)⁺ |
| 79 | 1 | 5-Me | -OH | -Me | HCl | NMR : 1.62 (3H, s), 2.66 (3H, s), 8.28 (1H, s). ; FAB : 296 (M+H) |
| 80 | 1 | 5-Et | -OH | -Me | HCl | NMR : 1.29 (3H, t, *J* = 7.3 Hz), 1.61 (3H,s), 8.26 (1H, d, *J* = 1.9 Hz). ; FAB : 310 (M+H)⁺ |
| 81 | 1 | 5-CF₃ | -OH | -Me | HCl | NMR : 1.67 (3H, s), 7.65 (1H, t, *J* = 7.8 Hz), 8.37 (1H, d, *J* = 1.4 Hz). ; FAB : 350 (M+H)⁺ |
| 82 | 1 | 8-Me | -OH | -Me | HCl | NMR : 1.69 (3H,s), 2.55 (3H,s) 8.23 (1H, d, *J* = 1.5 Hz). ; FAB :296 (M+H)⁺ |
| 83 | 1 | 5-Me, 8-Me | -OH | -Me | HCl | NMR: 1.67 (3H, s), 2.53 (3H, s), 2.67 (3H, s) ; FAB : 310 (M+H)⁺ |
| 84 | 2 | 5-F, 8-Me | -OH | -Me | HCl | NMR: 1.70 (3H, s), 2.53 (3H, s), 8.29 (1H, d, *J =* 1.5 Hz). ; FAB : 314 (M+H)⁺ |
| 85 | 2 | 5-F | -S(CH₂)₂S- | | HCl | NMR: 3.87 (1H, dt, *J* = 11.0, 3.9 Hz), 3.89 (1H, dt, *J* = 11.0, 5.8 Hz), 8.64 (1H, d, *J* = 1.5 Hz). ; FAB : 360 (M+H)⁺ |
| 86 | 1 | 5-F | -(CH₂)₂O(CH₂)₂- | | HCl | NMR : 1.74-1.80 (2H, m), 7.32 (1H, t, *J*=8.3 Hz), 8.54 (1H, s). ; FAB : 340(M+H)⁺ |
| 87 | 9 | 5-F | -OMe | -Me | HCl | NMR : 1.69 (3H, s), 2.67 (3H, s), 8.32 (1H, d, *J* = 1.5 Hz). ; FAB : 314(M+H)⁺ |
| 88 | 9 | 8-Me | -OMe | -Me | HCl | NMR : 1.72 (3H, s), 2.68 (3H, s), 8.22 (1H, d, *J=* 1.5 Hz). ; FAB : 310(M+H)⁺ |

**Table 15**

| | | | | | | |
|---|---|---|---|---|---|---|
| 89 | 9 | 5-F, 8-CH₂NMe₂ | -OMe | -Me | 2HCl | NMR : 1.81 (3H, s), 2.76 (3H, s), 8.42 (1H, s). ; FAB : 371 (M+H)⁺ |
| 90 | 9 | 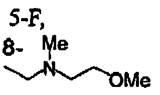 | -OMe | -Me | 2HCl | NMR : 1.81 and 1.82 (3H, s and s), 2.77 and 2.78 (3H, s and s), 2.84 and 2.85 (3H, s and s). ; FAB : 415 (M+H)⁺ |

**Table 16**

| | | | | | |
|---|---|---|---|---|---|
| 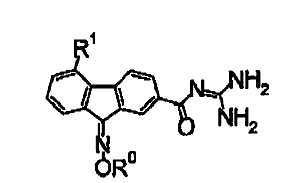 | | | | | |

| Ex | Syn | R⁰ | R¹ | Sal | Dat |
|---|---|---|---|---|---|
| 7 | 7 | -H | -CH₂OH | HCl | NMR : 4.91 and 4.93 (2H, s and s), 7.45-7.56 (2H, m), 7.63-7.74 (2H, m). ; FAB : 311 (M+H)⁺ |
| 91 | 9 | -H | -H | HCl | NMR : 7.51 (1H, dt, *J* = 7.3,1.0 Hz), 7.59 (1H, dt, *J* = 7.3, 1.0 Hz), 8.36 (1H, s). ; FAB : 281 (M+H)⁺ |
| 92 | 9 | -Me | -H | HCl | NMR : 4.24 and 4.25 (3H, sands), 7.44-7.65 (2H, m), 8.22-8.36 (2H, m Hz). ; FAB : 295(M+H)⁺ |
| 93 | 9 | -H | -F | | NMR : 7.29-7.61 (2H, m), 7.78-7.84 (1H, m), 8.18-8.29 (2H, m). ; FAB : 299 (M+H)⁺ |
| 94 | 7 | -H | -CH₂NMe₂ | HCl | NMR : 2.87 (6H, s), 4.83 (2H, s), 7.44-7.65 (1H, m). ; FAB : 338 (M+H)⁺ |
| 95 | 7 | -H | -CH₂OMe | HCl | NMR: 3.41 and 3.42 (3H, s and s), 4.82 and 4.84 (2H, s and s), 7.43-7.60 (2H, m). ; FAB: 325 (M+H)⁺ |

**Table 17**

| | | | | | |
|---|---|---|---|---|---|
| 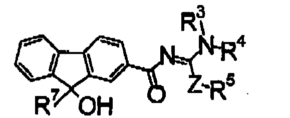 | | | | | |

| Ex | Syn | R⁷ | 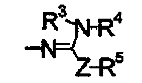 | Sal | Dat |
|---|---|---|---|---|---|
| 8 | 8 | H | 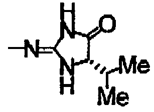 | | NMR : 0.94-0.99 (6H, m), 4.20 (1H, d, *J* = 4.0 Hz), 5.54 (1H, s) ; FAB: 350 (M+H)⁺ |
| 96 | 2 | Me | 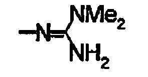 | HCl | NMR : 1.63 (3H, s), 3.18 (6H,s), 8.16 (1H, s) ; FAB : 310 (M+H)⁺ |
| 97 | 2 | Me | 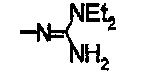 | HCl | NMR: 1.21 (6H, t, *J*= 6.8 Hz), 1.63 (3H, s), 8.16 (1H, s); FAB : 338 (M+H)⁺ |
| 98 | 2 | Me | 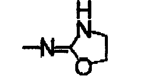 | | NMR : 1.59 (3H, s), 3.80 (2H, t, *J* = 8.8 Hz), 8.24 (1H, brs); FAB : 309 (M+H)⁺ |
| 99 | 2 | Me | 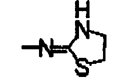 | HCl | NMR : 1.61 (3H, s), 3.42 (2H, t, *J* = 8.8 Hz), 8.27 (1H, brs). ; FAB : 325 (M+H)⁺ |
| 100 | 2 | Me | 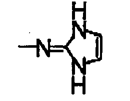 | | NMR : 1.62 (3H, s), 6.83 (2H, s), 8.26 (1H, d, *J* = 0.8Hz). ; FAB : 306 (M+H)⁺ |
| 101 | 2 | Me | 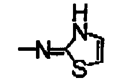 | | NMR : 1.64 (3H, s), 7.29 (1H, d, *J*= 3.6 Hz), 8.31 (1H, d, *J* = 1.6 Hz).; FAB : 323 (M+H)⁺ |
| 102 | 2 | Me | 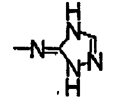 | | NMR: 1.61 (3H, s), 7.91 (1H, d, *J* = 7.6 Hz), 8.23 (1H, d, *J* = 1.6 Hz).; FAB : 307 (M+H)⁺ |
| 103 | 2 | Me | 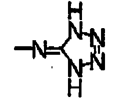 | | NMR : 1.64 (3H, s), 7.94 (1H, d, *J* = 7.8 Hz), 8.23 (1H, d, *J* = 1.6 Hz).; FAB : 308 (M+H)⁺ |
| 104 | 2 | Me | -NH₂ | | NMR : 1.60 (3H, s), 7.90 (1H, dd, *J*= 8.0, 1.6 Hz), 8.07 (1H, d, *J* = 1.2 Hz).; FAB : 240 (M+H)⁺ |

**Table 18**

| Ex | Syn | Str | Sal | Dat |
|---|---|---|---|---|
| 105 | 2 | 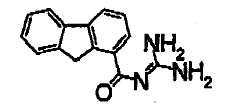 | HCl | NMR : 4.26 (2H, s), 7.36-7.46 (2H, m), 8.25 (1H, d, *J* = 7.8 Hz). ; FAB : 252 (M+H)⁺ |
| 106 | 2 | 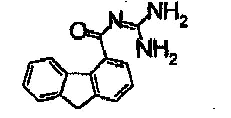 | HCl | NMR : 4.01 (2H, s), 7.36-7.44 (2H, m), 7.83-7.91 (1H, m). ; FAB : 252 (M+H)⁺ |
| 107 | 2 | 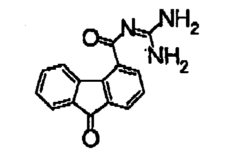 | | NMR : 7.33-7.39 (2H, m), 7.75 (1H, dd, J= 8.0, 1.2 Hz), 8.04 (1H, d, *J* = 7.6 Hz). ; FAB : 266 (M+H)⁺ |
| 108 | 2 | 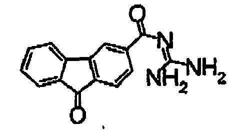 | HCl | NMR: 7.47 (1H, t, *J* = 7.4 Hz), 7.68-7.73 (2H, m), 8.67 (1H, s). ; FAB : 266 (M+H)⁺ |
| 109 | 3 | 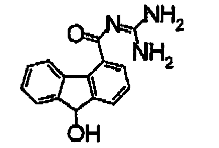 | HCl | NMR : 5.51 (1H, s), 7.35-7.44 (2H, m), 7.83 (1H, d, *J* = 7.1 Hz). ; FAB : 268 (M+H)⁺ |
| 110 | 3 | 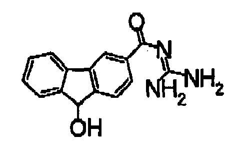 | HCl | NMR: 5.59 (1H, s), 7.39 (1H, t, *J*= 7.4 Hz), 8.74 (1H, s). ; FAB : 268 (M+H)⁺ |

**Table 19**

| | | | | | |
|---|---|---|---|---|---|
| 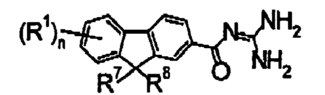 | | | | | |

| Cmp | 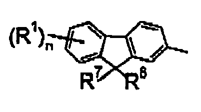 | Cmp | 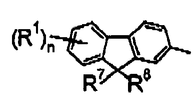 | Cmp | 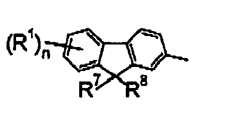 |
|---|---|---|---|---|---|
| 1 | 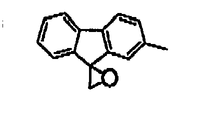 | 8 | 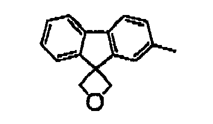 | 15 | 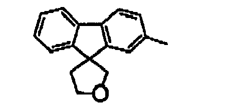 |
| 2 | 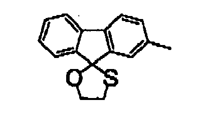 | 9 | 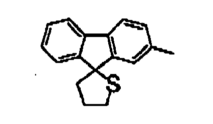 | 16 | 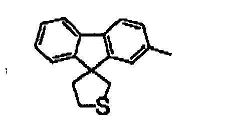 |
| 3 | 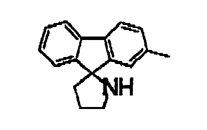 | 10 | 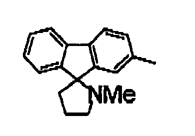 | 17 | 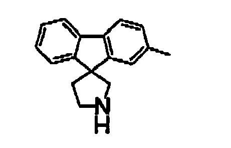 |
| 4 | 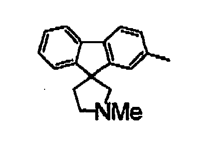 | 11 | 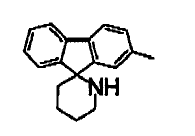 | 18 | 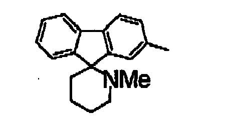 |
| 5 | 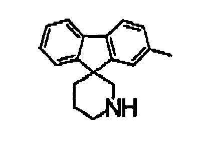 | 12 | 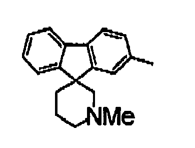 | 19 | 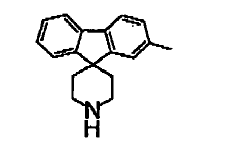 |
| 6 | 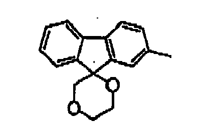 | 13 | 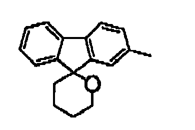 | 20 | 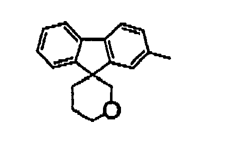 |
| 7 | 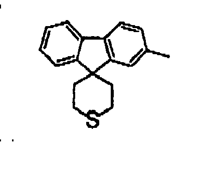 | 14 | 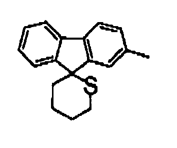 | 21 | 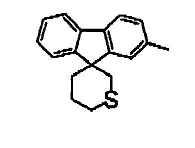 |

**Table 20**

| | | | | | |
|---|---|---|---|---|---|
| 22 | 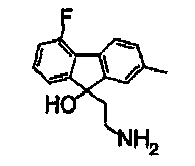 | 29 | 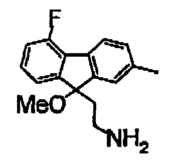 | 36 | 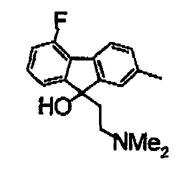 |
| 23 | 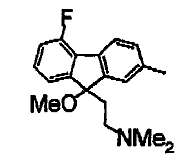 | 30 | 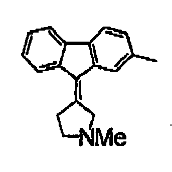 | 37 | 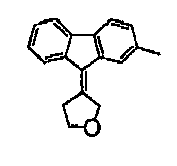 |
| 24 | 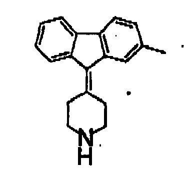 | 31 | 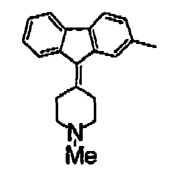 | 38 | 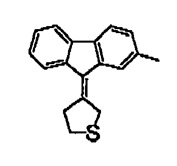 |
| 25 | 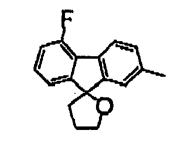 | 32 | 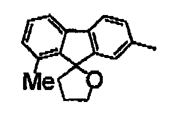 | 39 | 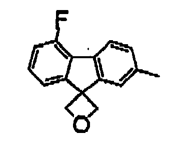 |
| 26 | 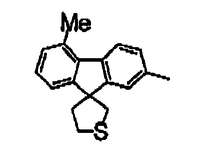 | 33 | 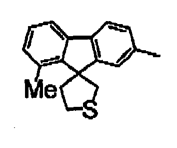 | 40 | 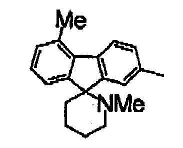 |
| 27 | 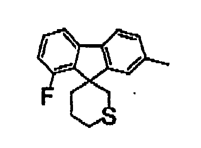 | 34 | 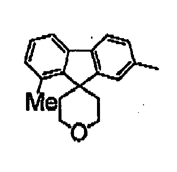 | 41 | 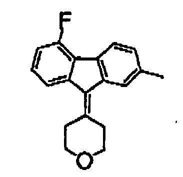 |
| 28 | 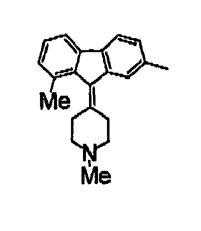 | 35 | 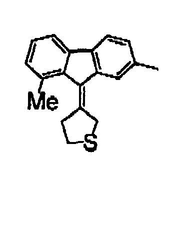 | 42 | 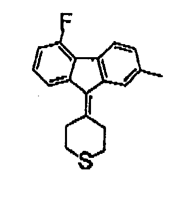 |

The prophylactic antimigraine agents of the invention will be illustrated by the following examples. The experimental results on the receptor affinity are shown in Reference Examples.

### Reference Example 1 Binding test of 5-HT_{2B} receptor

### (i) Preparation of membrane preparation

A human 5-HT_{2B} receptor expressing cell was prepared in accordance with a reference (FEBS Letters (1994) 342, 85 - 90). HEK293-ENBA cell was used as the gene transferring cell.

Cultured HEK293-EBNA cells expressing human 5-HT_{2B} receptor were washed with PBS(-). The cells were scraped in the presence of PBS(-), and the cells were recovered by centrifugation (1,000 rpm, 10 min, 4°C). They were homogenized using Polytron (PTA 10-TS) in the presence of 5 mM Tris-HCl (pH 7.4) buffer and centrifuged (40,000 x g, 10 min, 4°C). They were suspended using a homogenizer in the presence of 50 mM Tris-HCl (pH 7.4) buffer. They were subjected to centrifugation (40,000 x g, 10 min, 4°C), suspended in 50 mM Tris-HCl (pH 7.4) and stored at -80°C.

### (ii) Receptor binding test

A total volume of 500 µl containing 50 mM Tris-HCl-4 mM CaCl₂ (pH 7.4) buffer, the human 5-HT_{2B} receptor expressing HEK293-EBNA cell membrane preparation and a radio ligand [³H]Mesulergine (3.1 TBq/mmol) was incubated at 25°C for 1 hour. The compound was dissolved in 100% DMSO and diluted to respective concentrations. Nonspecific binding was defined as the binding quantity in the presence of 1 µM ritanserin, and the result of subtracting the nonspecific binding quantity from the total binding quantity was defined as the specific binding quantity. This was mixed with 4 ml of 50 mM Tris-HCl buffer (pH 7.4) and filtered under a reduced pressure using a GF/B glass filter, and the filter was washed (4 ml x 3) with the same buffer. The glass filter was soaked in 5 ml of a liquid scintillator (Aquasol-2) and the radioactivity was measured using a liquid scintillation counter. Concentration of the compound which inhibits 50% of the receptor binding, IC₅₀ value, was obtained by nonlinear regression analysis using SAS (ver. 6.11), and the Ki value which represents its affinity for the receptor was calculated using the formula of Cheng & Prussoff; Ki = IC₅₀/(1 + [L]/[Kd]) ([L]: ligand concentration, [Kd]: dissociation constant).

The compound of Preparation 3 which is described above showed a Ki value of 1.8 nM. Also, the compounds of Preparation 4, 7, 8, 34, 38, 56, 56a, 56b, 59, 60, 60a, 60b, 63, 71, 72, 77, 78a, 78b, 85 and 87 showed Ki values of from 0.1 to 350 nM.

### Reference Example 2 (2) Binding test of 5-HT₇ receptor

### (i) Preparation of membrane preparation

A human 5-HT₇ receptor expressing cell was prepared in accordance with references (J. Biol. Chem. (1993) 268, 31, 23422 - 23426, Br. J. Phaemacol. (1997) 122, 126-132). CHO cell was used as the gene transferring cell.

Cultured CHO cells expressing human 5-HT₇ receptor were washed with PBS(-). The cells were scraped in the presence of PBS(-), and the cells were recovered by centrifugation (1,000 rpm, 10 min, 4°C). They were homogenized using Polytron (PTA 10-TS) in the presence of 5 mM Tris-HCl (pH 7.4) buffer and centrifuged (40,000 x g, 10 min, 4°C). They were suspended using a homogenizer in the presence of 50 mM Tris-HCl (pH 7.4) buffer. They were subjected to centrifugation (40,000 x g, 10 min, 4°C), suspended in 50 mM Tris-HCl (pH 7.4) and stored at -80°C.

### (ii) Receptor binding test

A total volume of 500 µl containing 50 mM Tris-HCl-4 mM CaCl₂ (pH 7.4) buffer, the human 5-HT₇ receptor expressing CHO cell membrane preparation and a radio ligand [³H]5-HT (3.40 TBq/mmol) was incubated at 25°C for 1 hour., The compound was dissolved in 100% DMSO and diluted to respective concentrations. Nonspecific binding was defined as the binding quantity in the presence of 10 µM metergoline, and the result of subtracting the nonspecific binding quantity from the total binding quantity was defined as the specific binding quantity. This was mixed with 4 ml of 50 mM Tris-HCl buffer (pH 7.4) and filtered under a reduced pressure using a GF/B glass filter, and the filter was washed (4 ml x 3) with the same buffer. The glass filter was soaked in 5 ml of a liquid scintillator (Aquasol-2) and the radioactivity was measured using a liquid scintillation counter. Concentration of the compound which inhibits 50% of the receptor binding, IC₅₀ value, was obtained by nonlinear regression analysis using SAS (ver. 6.11), and the Ki value which represents its affinity for the receptor was calculated using the formula of Cheng & Prussoff; Ki = IC₅₀/(1 + [L]/[Kd]) ([L]: ligand concentration, [Kd]: dissociation constant).

The compound of Preparation 3 which is described above showed a Ki value of 17.6 nM. Also, the compounds of Preparation 4, 7, 8, 34, 38, 56, 56a, 56b, 59, 60, 60a, 60b, 63, 71, 72, 77, 78a, 78b, 85 and 87 showed Ki values of from 0.4 to 310 nM.

### Reference Example 3 Affinity for other receptors

Affinity of the compound of Preparation 3 for 5-HT_{1A}, 5-HT_{1B}, 5-HT_{2A}, 5-HT_{2C}, 5-HT₃, 5-HT₄, 5-HT₆, α₁, M₁ and D₂ receptors was verified using conventionally known techniques (Journal of Neurochemistry (1986) 47, 529 - 540; Molecular Pharmacology (1982) 21, 301 - 314; European Journal of Pharmacology (1985) 106, 539 - 546; Journal of Pharmacology Experimental Therapy (1992) 263, 1127 - 1132; British Journal of Pharmacology (1993) 109, 618 - 624; Molecular Pharmacology (1993) 43, 320 - 327; Molecular Pharmacology (1989) 35, 324 - 330; Cellular Molecular Neurobiology (1988) 8, 181 - 191; European Journal of Pharmacology (1988) 173, 177 - 182). As a result, IC₅₀ value of this compound was 1 µM or more on all of the 5-HT_{1A}, 5-HT_{1B}, 5-HT_{2A}, 5-HT_{2C}, 5-HT₃, 5-HT₄, 5-HT₆, α₁, M₁ and D₂ receptors. In addition, when the affinity for each of α₁, M₁ and D₂ receptors was verified using the aforementioned technique on the compounds of Preparation 56, 59, 60, 71, 72, 77 and 85 which are described above, 5-HT_{2B} and 5-HT₇ receptor selectivity of these compounds against α₁, M₁ and D₂ receptors was 100 times or more.

Based on the above results, it was shown that the compounds described in the Preparations have selective binding affinity for both of the 5-HT_{2B} and 5-HT₇ receptors.

In this connection, affinities of each of RS-127445 (2-amino-4-(4-fluoronaphth-1-yl)-6-isopropylpyrimidine; see WO 97/44326 for its production method) and SB-269970 ((R)-3-(2-(2-(4-methylpiperidin-1-yl)ethyl)pyrrolidine-1-sulfonyl)phenol; see WO 97/48681 for its production method) described in the following example 1 for respective receptors are conventionally known, and regarding the RS-127445, it has been reported that said compound has a pKi value of 9.5 for 5-HT_{2B} receptor, and is 1000 times more 5-HT_{2B} receptor selective against 5-HT_{1A}, 5-HT_{1B}, 5-HT_{2A}, 5-HT_{2C}, 5-HT₃, 5-HT₆, 5-HT₇, α₁, M₁ and D₂ receptors. Also, regarding the SB-269970, it has been reported, for example in J. Med Chen. (2000) 43, 342 - 345, that said compound has a pKi value of 8.9 for 5-HT_{2B} receptor, and is 250 times more 5-HT₇ receptor selective against 5-HT_{1A}, 5-HT_{1B}, 5-HT_{2A}, 5-HT_{2B}, 5-HT_{2C}, 5-HT₄, 5-HT-₆, α₁ and D₂ receptors.

### Example 1 Preventive effect in guinea pig migraine model

It has been suggested that an inflammatory protein leaked from the dural blood vessel caused by 5-HT is concerned in the onset of migraine. In this test system, migraine preventive effect was evaluated by measuring this leaked protein in the presence of a compound to be tested, and this was carried out by partially modifying the method described in Rachel A. Spokes and Vicki C. Middlefell, European Journal of Pharmacology (1995) 281, 75 - 79.

Hartley male guinea pig (250 - 350 g) was anesthetized by peritoneal administration (i.p.) of urethane (1.5 g/kg). By applying simple canulation to a saphena, 50 mg/kg of a fluorescent protein (FITC-BSA) was intravenously administered (i.v.) and 5 minutes thereafter, physiological saline or 1 µM of 5-HT was intravenously administered. By carrying out perfusion with physiological saline 15 minutes thereafter, blood was washed out. Each of RS-127445, SB-269970 and the compound of Preparation 3 was intraperitoneally administered, and other Preparation compounds orally, 30 minutes before administration of the fluorescent protein. By detaching the skull, dura mater was took out and incubated at 37°C for 16 hours in an Eppendorf tube in the presence of physiological saline of pH 11. Centrifugation was carried out, and the supernatant was dispensed into a plate. Fluorescence intensity was measured using a fluorescence plate reader (excitation wavelength 485 nm, absorption wavelength 530 nm). By measuring dura mater weight, fluorescence intensity per mg dural protein was calculated.

Values of the fluorescence intensity measured at the time of the administration of each compound and at the time of no administration are shown in Fig. 1 to Fig. 4. In each of them, the axis of abscissa shows dose of the compound, and the axis of ordinate fluorescence intensity per I mg dural blood vessel. The control indicates fluorescence intensity at the time of no addition of 5-HT, namely the reference value.

As shown in Fig. 1, the 5-HT_{2B} selective antagonistic compound RS-127445 showed an effect to reduce amount of the leaked protein at 3 mg/kg, but did not reduced to the reference value when the dose was increased from 3 mg/kg to 10 mg/kg.

In addition, as shown in Fig. 2, the 5-HT₇ selective antagonistic compound SB-269970 also showed the effect from 10 mg/kg, but did not reduced the amount of leaked protein to the reference value when the dose was increased from 3 mg/kg to 30 mg/kg.

On the other hand, as shown in Fig. 3, it was found that a synergistic effect is obtained when both compounds of RS-127445 and SB-269970 are simultaneously administered. That is, as shown in Fig. 1 and Fig. 2, it was shown that the minimum amount of both compounds showing maximum drug effect in this model is 3 mg/kg for RS-127445 and 10 mg/kg for SB-269970, but it was revealed that the amount of leaked protein is suppressed almost completely to the reference value when both compounds are simultaneously administered at the same dose. This result shows that when both functions of 5-HT_{2B} receptor and 5-HT₇ receptor are simultaneously inhibited, an excellent effect which cannot be attained by the selective inhibition of one of the receptors may be obtained.

This effect was the same when the compound of the invention simultaneously having selective 5-HT_{2B} receptor antagonism and 5-HT₇ receptor antagonism was used. That is, the compound of Preparation 3 which is described above almost completely suppressed the amount of leaked protein by 3 mg/kg of intraperitoneal administration as shown in Fig. 4.

In addition, the compounds of Preparation 60, 60a, 60b, 71, 72, 77, 78b, 85 and 87 which are described later also almost completely suppressed leakage of the protein by 10 mg/kg or 30 mg/kg of oral administration.

Based on the above results, it was shown that the prophylactic antimigraine agents of the present invention can completely inhibit leakage of the inflammatory protein by simultaneously having 5-HT_{2B} receptor antagonism and 5-HT₇ receptor antagonism. Accordingly, it is shown that the prophylactic antimigraine agents of the present invention has a possibility of effectively inhibiting inset of migraine and has excellent migraine preventive effect in comparison with one of the selective receptor antagonists.

### Industrial Applicability

The prophylactic antimigraine agents of the present invention exhibit a potent prophylactic effect for migraine by simultaneously inhibiting the function of the 5-HT_{2B} and 5-HT₇ receptors. In addition, the side effects caused by antagonism of other receptors than the 5-HT_{2B} and 5-HT₇ receptors, which side effects have been reported in the existing drugs, are reduced. Thus, they are useful as highly effective and highly safe prophylactic antimigraine agents.

## Claims

1. A prophylactic antimigraine agent comprising as an active ingredient a selective dual antagonist for the 5-HT_{2B} and 5-HT₇ receptors.

2. A prophylactic antimigraine agent as claimed in Claim 1, wherein the selective dual antagonist for the 5-HT_{2B} and 5-HT₇ receptors comprises a) a 5-HT_{2B} receptor antagonistic compound as a first ingredient having a selective binding affinity to the 5-HT_{2B} receptor, and b) a 5-HT₇ receptor antagonistic compound as a second ingredient having a selective binding affinity to the 5-HT₇ receptor.

3. A prophylactic antimigraine agent as claimed in Claim 1, wherein the selective dual antagonist for the 5-HT_{2B} and 5-HT₇ receptors comprises a dual antagonistic compound for the 5-HT_{2B} and 5-HT₇ receptors having a selective binding affinity to both of the 5-HT_{2B} and 5-HT₇ receptors.

4. A combined prophylactic preparation for migraine which comprises a) a first pharmaceutical preparation comprising as an active ingredient a 5-HT_{2B} receptor antagonistic compound having a selective binding affinity to the 5-HT_{2B} receptor, and b) a second pharmaceutical preparation comprising as an active ingredient a 5-HT₇ receptor antagonistic compound having a selective binding affinity to the 5-HT₇ receptor, and wherein the first and second preparations are administered simultaneously or separately.

5. A prophylactic antimigraine agent as claimed in Claim 1, wherein the binding affinity for the 5-HT_{2B} and 5-HT₇ receptors is respectively one-hundredth or less to the α₁, M₁, D₂, 5-HT_{1A}, 5-HT_{1B}, 5-HT_{2A}, 5-HT_{2C}, 5-HT₃, 5-HT₄ and 5-HT₆ receptors.

6. Use of the selective dual antagonist for the 5-HT_{2B} and 5-HT₇ receptors for the manufacture of a prophylactic antimigraine agent.

7. Use of "a 5-HT_{2B} receptor antagonistic compound having a selective binding affinity to the 5-HT_{2B} receptor" for the manufacture of a prophylactic antimigraine agent comprising as an active ingredient a selective dual antagonist for the 5-HT_{2B} and 5-HT₇ receptors.

8. Use of "a 5-HT₇ receptor antagonistic compound having a selective binding affinity to the 5-HT₇ receptor" for the manufacture of a prophylactic antimigraine agent comprising as an active ingredient a selective dual antagonist for the 5-HT_{2B} and 5-HT₇ receptors.

9. A method for prophylaxis of migraine which comprises administering a therapeutically effective amount of a selective dual antagonist for the 5-HT_{2B} and 5-HT₇ receptors to a patient.

10. A method for prophylaxis of migraine which comprises administering a combination comprising a pharmaceutical preparation containing as an active ingredient a 5-HT_{2B} selective receptor antagonistic compound and a pharmaceutical preparation containing as an active ingredient a 5-HT₇ receptor selective antagonistic compound, simultaneously or separately to a patient.
